# EUROPEAN PATENT APPLICATION

(11) **EP 3 289 873 A1**
(43) Date of publication of application: **07.03.2018**
(21) Application number: 16786566.6
(22) Date of filing: 28.04.2016
(51) Int. Cl.: A01N 1/02, A61K 9/08, A61K 31/472, A61K 31/4725, A61K 45/00, A61P 35/00, A61P 41/00, A61P 43/00

(54) **COMPOSITION HAVING COMPOUND ACCELERATING PHOSPHORYLATION OF AMPK AS EFFECTIVE COMPONENT**

(30) Priority: 30.04.2015 JP 2015093346
(71) Applicant: Kyoto University, Kyoto-shi, Kyoto 606-8501 (JP)
(72) Inventor: HAGIWARA Masatoshi, Kyoto-shi, Kyoto 606-8501 (JP); TOYOMOTO Masayasu, Kyoto-shi Kyoto 606-8501, (JP); KII Isao, Kyoto-shi Kyoto 606-8501 (JP); HOSOYA Takamitsu, Bunkyo-ku, Tokyo 113-8510 (JP); YOSHIDA Suguru, Bunkyo-ku, Tokyo 113-8510 (JP)
(74) Representative: Golding, Louise Ann
(86) International application number: PCT/JP2016/063381
(87) International publication number: WO 2016/175290

(57) **Abstract**

Provided is a composition that can be used for an organ preservation solution or perfusate. Alternatively, provided is a composition for prolonging the survival of normal cells and/or a composition for inhibiting the survival of cancer cells. In one or more embodiments, a composition containing, as an active component, a compound that enhances phosphorylation of AMPK (AMP-activated protein kinase). Said composition is one that can prolong the survival of cells (especially, normal cells). Furthermore, said composition is one that can inhibit the survival of cancer cells.

## Description

### [Technical Field]

The present disclosure relates to a compound that enhances phosphorylation of AMPK and a composition containing said compound as well as the use thereof. The present disclosure also relates to a composition that prolongs the survival of cells (especially, normal cells), a medical composition for inhibiting injury or cell death during perfusion, reperfusion, or preservation of a human or animal organ, tissue, or part thereof, and a pharmaceutical composition for inhibiting injury or cell death during perfusion or reperfusion of a human or animal organ, tissue, or part thereof. Furthermore, the present disclosure relates to a pharmaceutical composition for inducing programmed cell death in cancer cells, the pharmaceutical composition containing, as an active component, a compound that enhances phosphorylation of AMPK. Moreover, the present disclosure relates to a method of cancer prevention, amelioration, progression inhibition, and/or treatment as well as a method of inducing programmed cell death in cancer cells.

### [Background Art]

In organ transplant, the organ of a donor is not supplied with blood during the period from removal of said organ to its transplant to a recipient. Therefore, the absence of oxygen during the ischemic interval may cause cell injury or necrotic tissue change and eventually may impair the vital force and functionality of said organ. Furthermore, during reperfusion after myocardial infarction or cerebral infarction or reperfusion of a transplanted organ, ischemia-reperfusion syndrome may be caused by reperfusion stress.

In order to solve these problems as much as possible, many organ preservation solutions and perfusates have been developed so far. Examples thereof include Bretschneider solution; Bretschneider's HTK solution, trade name: Custodiol (registered trademark); Euro-Collins solution; UW solution (University of Wisconsin solution); St. Thomas'Hospital solution (Plegisol (registered trademark)); Viaspan (registered trademark) ("Belzer UW" DuPont-Pharma GmbH, Bad Homburg); Celsior (registered trademark) (Imtix Sangstat, Lyon); Perfadex (registered trademark) (Vitrolife AB, Gothenburg); Polysol (registered trademark); and ET-Kyoto solution.

Furthermore, Patent Document 1 discloses a cell or organ preservation solution containing, as an active component, an N-terminal acylated polypeptide with 5 amino acid residue length. Moreover, Patent Document 2 discloses solutions for perfusing and preserving organs, parts of organs, tissues, or parts of tissues of human or animal origin, the solution containing a stimulant/activator of soluble guanylate cyclase.

### [Prior Art Documents]

### [Patent Documents]

[Patent Document 1] JP2010-239963A
[Patent Document 2] JP2010-529053A

### [SUMMARY OF THE INVENTION]

### [Problem to be Solved by the Invention]

In an aspect, the present disclosure provides a composition that can be used for an organ preservation solution or perfusate.

In another aspect, the present disclosure provides a composition that exhibits a cell death inhibitory effect on normal cells or non-cancer cells and/or a composition that exhibits a cell death-inducing effect on cancer cells.

### [Means for Solving Problem]

In one or more embodiments, the present disclosure relates to a composition that prolongs the survival of cells, especially normal cells, the composition containing, as an active component, a compound that enhances phosphorylation of AMP-activated protein kinase (AMPK).

In one or more embodiments, the present disclosure relates to use of the composition according to the present disclosure as a preservation solution, perfusate, or reperfusate for a human or animal organ, tissue, or part thereof before, during, or after transplant thereof, or a modifier therefor.

In one or more embodiments, the present disclosure relates to use of the composition according to the present disclosure in production of a perfusate or preservation solution for a human or animal organ, tissue, or part thereof, for inhibiting injury during preservation, storage, or transportation of an ex vivo organ or tissue, for inhibiting reperfusion injury, for inhibiting graft ischemic injury, for improving functional recovery after transplant of an organ or tissue, or for inhibiting transplant failure.

In one or more embodiments, the present disclosure relates to a method of preserving, storing, or transporting an ex vivo human or animal organ, tissue, or part thereof, or a method for inhibiting injury during preservation, storage, or transportation thereof, the method including contacting the organ, tissue, or part thereof with the composition according to the present disclosure.

In one or more embodiments, the present disclosure relates to a pharmaceutical composition for inducing programmed cell death in cancer cells, the pharmaceutical composition containing, as an active component, a compound that enhances phosphorylation ofAMPK.

In one or more embodiments, the present disclosure relates to a method of cancer prevention, amelioration, progression inhibition, and/or treatment, the method including administering the pharmaceutical composition according to the present disclosure to a subject.

In one or more embodiments, the present disclosure relates to a method of inducing programmed cell death in cancer cells, the method including contacting the cancer cells with the compound that enhances phosphorylation of AMPK according to the present disclosure or administering, to a biological body with the cancer cells, the compound that enhances phosphorylation of AMPK according to the present disclosure, a prodrug thereof, or a pharmaceutically acceptable salt thereof.

### [Brief Description of Drawings]

[Fig. 1] Fig. 1 shows an example of the results obtained by detecting, by Western blotting, the abundance of phosphorylated AMPKα, total AMPKα, phosphorylated ACC, and total ACC at 10, 30, 60, 90, and 120 minutes after the addition of Compound 1.
[Fig. 2] FIG. 2 shows an example of graphs comparing the viable cell numbers of normal cells in the serum-starved condition in the presence and absence of Compound 1.
[Fig. 3] Fig. 3 shows examples of graphs comparing the viable cell numbers of primary cultured cells in the serum-starved condition in the presence and absence of Compound 1.
[Fig. 4] Fig. 4 shows examples of graphs comparing the inhibitory effect of Compound 1 on the increase in liver deviation enzymes AST and ALT in a rat liver warm ischemia reperfusion model, with a positive control AICAR and a negative control Vehicle.
[Fig. 5] Fig. 5 shows examples of graphs comparing the viable cell numbers of cancer cells (HeLa and Jurkat cells) in the presence and absence of Compound 1.
[Fig. 6] Fig. 6 shows an example of the results of detecting, using an autophagy inhibitor (BafA), the autophagy marker (LC3-II) in cancer cells in which cell death was induced by Compound 1.
[Fig. 7] Fig. 7 shows an example of the results of confirming the effect of an autophagy inhibitor (3-MA) on cancer cells in which cell death is induced by Compound 1.

### [Description of Preferred Embodiments]

### [Composition that prolongs the survival of cells]

In an aspect, the present disclosure is based on the finding that a compound that enhances phosphorylation ofAMPK (AMP-activated protein kinase) can prolong the survival of non-cancer cells. In one or more embodiments, the "phosphorylation of AMPK" denotes the phosphorylation of the α-subunit Thr172 or denotes the phosphorylation of Thr/Ser at the site homologous thereto. In one or more embodiments, cells refer to human or non-human animal cells. In the present disclosure, the term "cells" denote normal cells or non-cancer cells unless otherwise specified. In one or more embodiments, the animal is a mammal. In one or more embodiments, the phrase "prolong the survival of cells" in the present disclosure includes inhibiting cell death or inhibiting the reduction in the viable cell number during culture. In one or more further embodiments, the expression "prolong the survival of cells" includes inhibiting cell death in serum-free culture or ischemia or inhibiting cell death in the primary culture.

Furthermore, in one or more embodiments, the enhancement of phosphorylation of AMPK in the present disclosure may be the enhancement of phosphorylation of AMPK mediated by a G protein-coupled receptor (GPCR).

Accordingly, in an aspect, the composition according to the present disclosure is a composition containing, as an active component, a compound that enhances phosphorylation ofAMPK. The composition according to the present disclosure can prolong the survival of cells, especially normal cells.

In another aspect, the present disclosure is based on the finding that the composition according to the present disclosure can be used for a preservation solution and/or perfusate for organs, etc. In one or more non-limiting embodiments, the composition according to the present disclosure can inhibit injury (cell death), which can be induced by ischemia, of a human or animal organ, tissue, or part thereof or can prolong the ischemic interval thereof, in the form of a preservation solution. Furthermore, in one or more non-limiting embodiments, the composition according to the present disclosure can inhibit injury (cell death), which can be induced by ischemia, of a human or animal organ, tissue, or part thereof or can inhibit or avoid ischemia-reperfusion injury, in the form of a perfusate.

In the present disclosure, the preservation temperature for the "preservation of a human or animal organ, tissue, or part thereof" is not particularly limited and the preservation may include preservation in a frozen state, in a refrigerated state, and/or in a normal temperature state.

Therefore, in one or more non-limiting embodiments, the composition according to the present disclosure can be used as a preservation solution for organs, etc. that are used for transplant. Furthermore, in one or more non-limiting embodiments, the composition according to the present disclosure can be used as a perfusate after transplant or after cerebral infarction or myocardial infarction.

In one or more non-limiting embodiments, the preservation solution and the perfusate each are in the form of a liquid mixture or a solution that contains a preservation solution and/or perfusate for organs, etc., which has been used conventionally or will be used in future, and a compound that enhances phosphorylation of AMPK. In one or more embodiments, examples of the preservation solution and/or perfusate may include UW solution, Bretschneider solution, Bretschneider's HTK solution, Euro-Collins solution, St. Thomas' Hospital solution, ET-Kyoto solution, plasma, serum, blood, and combinations thereof.

In still another aspect, the present disclosure is based on the finding that the composition according to the present disclosure can be used for the modification of a preservation solution and/or perfusate for organs, etc. In one or more non-limiting embodiments, the composition according to the present disclosure is added, in the form of an additive, to a preservation solution and/or perfusate for organs, etc. In one or more non-limiting embodiments, the preservation solution containing the composition according to the present disclosure added thereto can inhibit injury (cell death), which can be induced by ischemia, of a human or animal organ, tissue, or part thereof or can prolong the ischemic interval thereof. In one or more non-limiting embodiments, the perfusate containing the composition according to the present disclosure added thereto can inhibit injury (cell death), which can be induced by ischemia, of a human or animal organ, tissue, or part thereof or can inhibit or avoid ischemia-reperfusion injury.

### [Medical Composition]

In one or more non-limiting embodiments, the composition according to the present disclosure is a medical composition for inhibiting injury or cell death during perfusion, reperfusion, or preservation of a human or animal organ, tissue, or part thereof. The medical composition according to this embodiment can be used as a preservation solution for organs, etc. that are used for transplant or as an additive for said preservation solution in one or more non-limiting embodiments and can inhibit injury (cell death), which can be induced by ischemia, of a human or animal organ, tissue, or part thereof or can prolong the ischemic interval thereof in one or more embodiments.

### [Pharmaceutical Composition]

In one or more non-limiting embodiments, the composition according to the present disclosure is a pharmaceutical composition for inhibiting injury or cell death during perfusion or reperfusion of a human or animal organ, tissue, or part thereof. The pharmaceutical composition according to this embodiment can be used as a perfusate after transplant or after cerebral infarction or myocardial infarction or as an additive for said perfusate in one or more non-limiting embodiments, and it can inhibit injury (cell death), which can be induced by ischemia, of a human or animal organ, tissue, or part thereof or can inhibit or avoid ischemia-reperfusion injury in one or more embodiments.

### [Preservation Solution and Perfusate]

In one or more non-limiting embodiments, the composition according to the present disclosure is a preservation solution or perfusate for inhibiting injury or cell death during perfusion, reperfusion, or preservation of a human or animal organ, tissue, or part thereof. The preservation solution or perfusate according to the present disclosure is composed of a compound that enhances phosphorylation of AMPK and a base solution, with the compound being dissolved or mixed in the base solution. The base solution is a preservation solution/or perfusate for organs, etc. that has been used conventionally or will be used in future in one or more embodiments, and examples thereof may include UW solution, Bretschneider solution, Bretschneider's HTK solution, Euro-Collins solution, St. Thomas'Hospital solution, ET-Kyoto solution, plasma, serum, blood, and combinations thereof in one or more embodiments.

In one or more non-limiting embodiments, the concentration of the compound that enhances phosphorylation of AMPK in the preservation solution or perfusate according to the present disclosure is a concentration that can enhance the phosphorylation of AMPK, for example, 2 to 150 µM.

### [Additive]

In one or more non-limiting embodiments, the composition according to the present disclosure is an additive for modifying a preservation solution or a perfusate. The preservation solution or perfusate to be modified by the additive according to the present disclosure is a preservation solution/or perfusate for organs, etc. that has been used conventionally or will be used in future in one or more embodiments, and examples thereof may include UW solution, Bretschneider solution, Bretschneider's HTK solution, Euro-Collins solution, St. Thomas' Hospital solution, ET-Kyoto solution, plasma, serum, blood, and combinations thereof in one or more embodiments. In one or more non-limiting embodiments, the preservation solution containing the additive according to the present disclosure added thereto can inhibit injury (cell death), which can be induced by ischemia, of a human or animal organ, tissue, or part thereof or can prolong the ischemic interval thereof. In one or more non-limiting embodiments, the perfusate containing the additive according to the present disclosure added thereto can inhibit injury (cell death), which can be induced by ischemia, of a human or animal organ, tissue, or part thereof or can inhibit or avoid ischemia-reperfusion injury. In one or more non-limiting embodiments, the additive according to the present disclosure can be added in such an amount that the concentration of the compound that enhances phosphorylation of AMPK in the preservation solution or perfusate, to which the additive has been added, can enhance the phosphorylation of AMPK, or in such an amount that results in a concentration of 2 to 150 µM.

### [Compound that enhances phosphorylation of AMPK]

In one or more non-limiting embodiments, the compound that enhances phosphorylation of AMPK, which is the active component of the composition according to the present disclosure, is a compound represented by Formula (I) below or a prodrug thereof, or a pharmaceutically acceptable salt thereof.

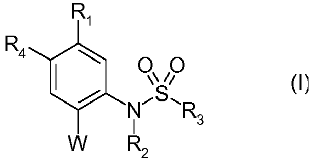

In Formula (I), R₁ represents a hydrogen atom, substituted or unsubstituted C₁₋₆ alkyl group, substituted or unsubstituted C₂₋₆ alkenyl group, substituted or unsubstituted C₂₋₆ alkynyl group, substituted or unsubstituted C₆₋₁₀ aryl group, halogen atom, nitro group, cyano group, azide group, hydroxy group, substituted or unsubstituted C₁₋₆ alkoxy group, substituted or unsubstituted C₁₋₆ alkylthio group, substituted or unsubstituted C₁₋₆ alkylsulfonyl group, carboxy group, formyl group, substituted or unsubstituted C₁₋₆ alkoxycarbonyl group, acyl group, acylamino group, or sulfamoyl group;
R₂ represents a hydrogen atom, substituted or unsubstituted C₁₋₆ alkyl group, or substituted or unsubstituted aryl group;
R₃ represents a substituted or unsubstituted C₁₋₆ alkyl group, substituted or unsubstituted C₂₋₆ alkenyl group, substituted or unsubstituted C₆₋₁₀ aryl group, substituted or unsubstituted nitrogen-containing heterocyclic ring, or substituted or unsubstituted fused aromatic heterocyclic ring;
R₄ represents a hydrogen atom or a halogen atom; and
W represents a hydrogen atom, substituted or unsubstituted C₁₋₆ alkyl group, substituted or unsubstituted C₆₋₁₀ aryl group, halogen atom, hydroxy group, substituted or unsubstituted C₁₋₆ alkoxy group, substituted or unsubstituted C₁₋₆ alkylthio group, substituted or unsubstituted nitrogen-containing heterocyclic ring, or substituted or unsubstituted fused aromatic heterocyclic ring, or W is represented by Formula (II) below.

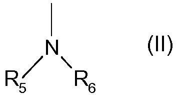

In Formula (II), R₅ and R₆, which are identical to or different from each other, each represent a hydrogen atom, substituted or unsubstituted C₁₋₆ alkyl group, substituted or unsubstituted nitrogen-containing heterocyclic ring, substituted or unsubstituted fused aromatic heterocyclic ring, acyl group, or acylamino group; or R₅ and R₆ together with the adjacent nitrogen atom each form a substituted or unsubstituted heterocyclic ring or a substituted or unsubstituted fused aromatic heterocyclic ring; or
R₅ and R₆ each represent a substituted or unsubstituted cycloalkylideneamino group or a substituted or unsubstituted aromatic ring-fused cycloalkylidene group.

From the viewpoint of use for an organ preservation solution or perfusate, in Formula (1), R₁ is preferably a halogen-substituted C₁₋₃ alkyl group, more preferably a trifluoromethyl group, R₂ is preferably a hydrogen atom, R₃ is a phenyl group, halogen-substituted or C₁₋₃ alkyl-substituted phenyl group, naphthyl group, halogen-substituted or C₁₋₃ alkyl-substituted naphthyl group, nitrogen-containing heterocyclic ring, or halogen-substituted or C₁₋₃ alkyl-substituted nitrogen-containing heterocyclic ring, R₄ is preferably a hydrogen atom or a halogen atom, and W is preferably the following:

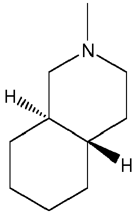

Therefore, the compound represented by Formula (I) is a compound represented by Formula (III) in one or more non-limiting embodiments.

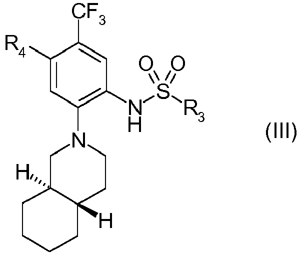

In Formula (III), R₃ represents a substituted or unsubstituted C₁₋₆ alkyl group, substituted or unsubstituted C₂₋₆ alkenyl group, substituted or unsubstituted C₆₋₁₀ aryl group, substituted or unsubstituted nitrogen-containing heterocyclic ring, or substituted or unsubstituted fused aromatic heterocyclic ring, and R₄ represents a hydrogen atom or a halogen atom. From the viewpoint of use for an organ preservation solution or perfusate, R₃ is preferably a phenyl group, halogen-substituted or C₁₋₃ alkyl-substituted phenyl group, naphthyl group, halogen-substituted or C₁₋₃ alkyl-substituted naphthyl group, nitrogen-containing heterocyclic ring, or halogen-substituted or C₁₋₃ alkyl-substituted nitrogen-containing heterocyclic ring.

In the present disclosure, in one or more embodiments, examples of the C₁₋₆ alkyl group include linear or branched alkyl groups such as a methyl group, ethyl group, 1-propyl group, 2-propyl group, 2-methyl-1-propyl group, 2-methyl-2-propyl group, 1-butyl group, 2-butyl group, 1-pentyl group, 2-pentyl group, 3-pentyl group, 2-methyl-1-butyl group, 3-methyl-1-butyl group, 2-methyl-2-butyl group, 3-methyl-2-butyl group, 2,2-dimethyl-1-propyl group, 1-hexyl group, 2-hexyl group, 3-hexyl group, 2-methyl-1-pentyl group, 3-methyl-1-pentyl group, 4-methyl-1-pentyl group, 2-methyl-2-pentyl group, 3-methyl-2-pentyl group, 4-methyl-2-pentyl group, 2-methyl-3-pentyl group, 3-methyl-3-pentyl group, 2,3-dimethyl-1-butyl group, 3,3-dimethyl-1-butyl group, 2,2-dimethyl-1-butyl group, 2-ethyl-1-butyl group, 3,3-dimethyl-2-butyl group, and 2,3-dimethyl-2-butyl group, as well as cyclic alkyl groups such as cyclopropyl, cyclobutyl, cyclopentyl, and cyclohexyl.

In the present disclosure, in one or more embodiments, the C₂₋₆ alkenyl group denotes a linear or branched alkenyl group having 2 to 6 carbon atoms, and specific examples thereof include a vinyl group, allyl group, 1-propenyl group, 2-propenyl group, 1-butenyl group, 2-butenyl group, 3-butenyl group, pentenyl group, and hexenyl group.

In the present disclosure, in one or more embodiments, the C₂₋₆ alkynyl group denotes a linear or branched alkynyl group having 2 to 6 carbon atoms, and specific examples thereof include an ethynyl group, 1-propynyl group, 2-propynyl group, butynyl group, pentynyl group, and hexynyl group.

In the present disclosure, in one or more embodiments, the C₁₋₆ alkoxy group denotes an oxy group to which the above-defined C₁₋₆ alkyl group is bonded, and specific examples thereof include a methoxy group, ethoxy group, 1-propyloxy group, 2-propyloxy group, 2-methyl-1-propyloxy group, 2-methyl-2-propyloxy group, 1-butyloxy group, 2-butyloxy group, 1-pentyloxy group, 2-pentyloxy group, 3-pentyloxy group, 2-methyl-1-butyloxy group, 3-methyl-1-butyloxy group, 2-methyl-2-butyloxy group, 3-methyl-2-butyloxy group, 2,2-dimethyl-1-propyloxy group, 1-hexyloxy group, 2-hexyloxy group, 3-hexyloxy group, 2-methyl-1-pentyloxy group, 3-methyl-1-pentyloxy group, 4-methyl-1-pentyloxy group, 2-methyl-2-pentyloxy group, 3-methyl-2-pentyloxy group, 4-methyl-2-pentyloxy group, 2-methyl-3-pentyloxy group, 3-methyl-3-pentyloxy group, 2,3-dimethyl-1-butyloxy group, 3,3-dimethyl-1-butyloxy group, 2,2-dimethyl-1-butyloxy group, 2-ethyl-1-butyloxy group, 3,3-dimethyl-2-butyloxy group, and 2,3-dimethyl-2-butyloxy group.

In the present disclosure, in one or more embodiments, the C₁₋₆ alkylthio group denotes a thio group to which the above-defined C₁₋₆ alkyl group is bonded, and specific examples thereof include a methylthio group, ethylthio group, 1-propylthio group, 2-propylthio group, butylthio group, and pentylthio group.

In the present disclosure, in one or more embodiments, the C₁₋₆ alkylsulfonyl group denotes a sulfonyl group to which the above-defined C₁₋₆ alkyl group is bonded, and specific examples thereof include a methylsulfonyl group, ethylsulfonyl group, 1-propylsulfonyl group, and 2-propylsulfonyl group.

In the present disclosure, in one or more embodiments, the C₁₋₆ alkoxycarbonyl group denotes a carbonyl group to which the above-defined C₁₋₆ alkyl group is bonded, and specific examples thereof include a methoxycarbonyl group, ethoxycarbonyl group, 1-propyloxycarbonyl group, and 2-propyloxycarbonyl group.

In the present disclosure, in one or more embodiments, the C₆₋₁₀ aryl group denotes an aromatic hydrocarbon cyclic group having 6 to 10 carbon atoms, and specific examples thereof include a phenyl group, 1-naphthyl group, and 2-naphthyl group.

In the present disclosure, the "heterocyclic ring" contains 1 to 2 hetero atoms in the atoms constituting the ring and may contain a double bond in the ring, and it denotes a non-aromatic ring or an aromatic ring. In the present disclosure, the "hetero atom" denotes a sulfur atom, oxygen atom, or nitrogen atom. In the present disclosure, the heterocyclic ring may be a fused heterocyclic ring in which two or more rings are fused.

In the present disclosure, the "nitrogen-containing heterocyclic ring" contains 1 to 2 nitrogen atoms in the atoms constituting the ring and may contain a double bond in the ring, and it denotes a non-aromatic ring or an aromatic ring. When the nitrogen-containing heterocyclic ring is a fused heterocyclic ring, the nitrogen atom only needs to be present in at least one ring.

In the present disclosure, the substituent may be one or a plurality of substituents that are identical to or different from each other, and in one or more embodiments, examples thereof include a halogen atom, cyano group, trifluoromethyl group, nitro group, hydroxy group, methylenedioxy group, C₁₋₆ alkyl group, C₁₋₆ alkoxy group, benzyloxy group, C₁₋₆ alkanoyloxy group, amino group, mono-C₁₋₆ alkylamino group, di-C₁₋₆ alkylamino group, carbamoyl group, C₁₋₆ alkylaminocarbonyl group, di-C₁₋₆ alkylaminocarbonyl group, carboxyl group, C₁₋₆ alkoxycarbonyl group, C₁₋₆ alkylthio group, C₁₋₆ alkylsulfinyl group, C₁₋₆ alkylsulfonyl group, C₁₋₆ alkanoylamino group, or C₁₋₆ alkylsulfonamide group. In the present disclosure, in one or more embodiments, examples of the halogen atom include fluorine, chlorine, bromine, and iodine atoms.

Furthermore, when an asymmetric carbon atom is present and/or when a stereoisomer is present, the compounds represented by Formulae (I) and (III) each are a mixture or isolate of the respective isomers in one or more embodiments.

In the present disclosure, in one or more embodiments, examples of the "prodrug" include those that are readily hydrolyzed in vivo to regenerate the compound represented by Formula (I), and include, in the case of, for example, a compound having a carboxyl group, a compound in which the carboxyl group is converted to an alkoxycarbonyl group, a compound in which the carboxyl group is converted to an alkylthiocarbonyl group, and a compound in which the carboxyl group is converted to an alkylaminocarbonyl group. Furthermore, in the case of a compound having an amino group, examples of the prodrug include a compound in which the amino group is substituted with an alkanoyl group to form an alkanoylamino group, a compound in which the amino group is substituted with an alkoxycarbonyl group to form an alkoxycarbonylamino group, a compound in which the amino group is converted to an acyloxymethylamino group, and a compound in which the amino group is converted to hydroxylamine. Furthermore, in the case of a compound having a hydroxy group, examples of the prodrug include a compound in which the hydroxy group is substituted with an acyl group to form an acyloxy group, a compound in which the hydroxy group is converted to a phosphate ester, and a compound in which the hydroxy group is converted to an acyloxymethyloxy group. Examples of the alkyl moiety of each group that is used for such prodrug conversion include the above-mentioned alkyl groups, and the alkyl groups may be substituted (with, for example, an alkoxy group having 1 to 6 carbon atoms). In one or more embodiments, in the case of, for example, a compound in which the carboxyl group is converted to an alkoxycarbonyl group, examples of the alkyl moiety include lower (for example, C₁₋₆) alkoxycarbonyl such as methoxycarbonyl or ethoxycarbonyl as well as lower (for example, C₁₋₆) alkoxycarbonyl substituted with an alkoxy group such as methoxymethoxycarbonyl, ethoxymethoxycarbonyl, 2-methoxyethoxycarbonyl, 2-methoxyethoxymethoxycarbonyl, or pivaloyloxymethoxycarbonyl.

In the present disclosure, the "pharmaceutically acceptable salt" includes a pharmaceutically, pharmacologically, and/or medically acceptable salt, and examples thereof include inorganic acid salts, organic acid salts, inorganic base salts, organic base salts, and acidic or basic amino acid salts.

Preferred examples of the inorganic acid salts include hydrochloride, hydrobromate, sulfate, nitrate, and phosphate. Preferred examples of the organic acid salts include acetate, succinate, fumarate, maleate, tartrate, citrate, lactate, stearate, benzoate, methanesulfonate, and p-toluenesulfonate.

Preferred examples of the inorganic base salts includes alkali metal salts such as sodium salt and potassium salt, alkaline earth metal salts such as calcium salt and magnesium salt, aluminum salt, and ammonium salt. Preferred examples of the organic base salts include diethylamine salt, diethanolamine salt, meglumine salt, and N, N'-dibenzylethylenediamine salt.

Preferred examples of the acidic amino acid salts include aspartate and glutamate. Preferred examples of the basic amino acid salts include arginine salt, lysine salt, and ornithine salt.

In the present disclosure, the "salt of the compound" may include a hydrate that can be formed by leaving the compound to stand in the atmosphere to absorb moisture. Furthermore, in the present disclosure, the "salt of the compound" may also include a solvate that can be formed with the compound absorbing some other type of solvent.

In one or more non-limiting embodiments, examples of the compound represented by Formula (I) include compounds of the following formulae.

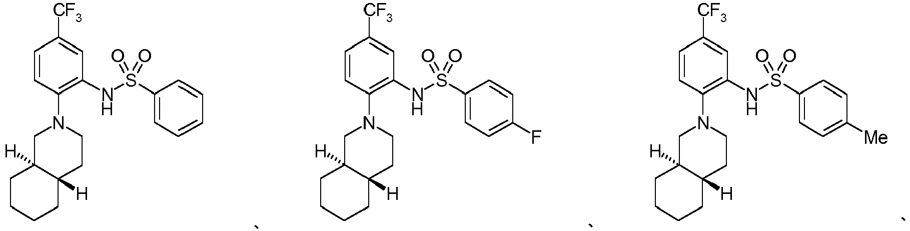

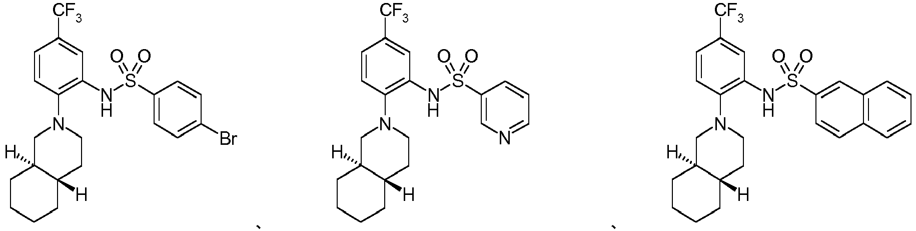

In one or more embodiments, the composition according to the present disclosure may be a liquid in the form of a preservation solution and/or a perfusate, a concentrate thereof, or an additive or may be a solid in the form of an additive for a preservation solution and/or a perfusate.

Accordingly, the present disclosure may further relate to one or more of the following embodiments.
[A1] A composition that prolongs the survival of cells, the composition containing, as an active component, a compound that enhances phosphorylation of AMPK.
[A2] The composition according to [A1], wherein the composition is a medical composition for inhibiting injury or cell death during perfusion, reperfusion, or preservation of a human or animal organ, tissue, or part thereof.
[A3] The composition according to [A1], wherein the composition is a pharmaceutical composition for inhibiting injury or cell death during perfusion or reperfusion of a human or animal organ, tissue, or part thereof.
[A4] The composition according to any one of [A1] to [A3], wherein the composition is an organ preservation solution or an organ perfusate in which the compound that enhances phosphorylation of AMPK is dissolved or mixed in a base solution, and the base solution is selected from the group consisting of UW solution, Bretschneider solution, Bretschneider's HTK solution, Euro-Collins solution, St. Thomas' Hospital solution, ET-Kyoto solution, plasma, serum, blood, and combinations thereof.
[A5] The composition according to any one of [A1] to [A4], wherein the composition is an additive for modifying an organ preservation solution or an organ perfusate.
[A6] The composition according to [A5], wherein the organ preservation solution or the organ perfusate is selected from the group consisting of UW solution, Bretschneider solution, Bretschneider's HTK solution, Euro-Collins solution, St. Thomas' Hospital solution, ET-Kyoto solution, plasma, serum, blood, and combinations thereof.
[A7] The composition according to any one of [A1] to [A6], wherein the compound that enhances phosphorylation of AMPK is a compound represented by Formula (I) below or a prodrug thereof, or a pharmaceutically acceptable salt thereof 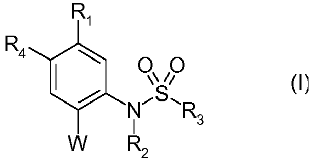 where R₁ represents a hydrogen atom, substituted or unsubstituted C₁₋₆ alkyl group, substituted or unsubstituted C₂₋₆ alkenyl group, substituted or unsubstituted C₂₋₆ alkynyl group, substituted or unsubstituted C₆₋₁₀ aryl group, halogen atom, nitro group, cyano group, azide group, hydroxy group, substituted or unsubstituted C₁₋₆ alkoxy group, substituted or unsubstituted C₁₋₆ alkylthio group, substituted or unsubstituted C₁₋₆ alkylsulfonyl group, carboxy group, formyl group, substituted or unsubstituted C₁₋₆ alkoxycarbonyl group, acyl group, acylamino group, or sulfamoyl group;
   R₂ represents a hydrogen atom, substituted or unsubstituted C₁₋₆ alkyl group, or substituted or unsubstituted aryl group;
   R₃ represents a substituted or unsubstituted C₁₋₆ alkyl group, substituted or unsubstituted C₂₋₆ alkenyl group, substituted or unsubstituted C₆₋₁₀ aryl group, substituted or unsubstituted nitrogen-containing heterocyclic ring, or substituted or unsubstituted fused aromatic heterocyclic ring;
   R₄ represents a hydrogen atom or a halogen atom; and
   W represents a hydrogen atom, substituted or unsubstituted C₁₋₆ alkyl group, substituted or unsubstituted C₆₋₁₀ aryl group, halogen atom, hydroxy group, substituted or unsubstituted C₁₋₆ alkoxy group, substituted or unsubstituted C₁₋₆ alkylthio group, substituted or unsubstituted nitrogen-containing heterocyclic ring, or substituted or unsubstituted fused aromatic heterocyclic ring, or W is represented by General Formula (II) below: 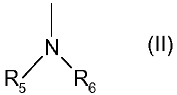 where R₅ and R₆, which are identical to or different from each other, each represent a hydrogen atom, substituted or unsubstituted C₁₋₆ alkyl group, substituted or unsubstituted nitrogen-containing heterocyclic ring, substituted or unsubstituted fused aromatic heterocyclic ring, acyl group, or acylamino group; or R₅ and R₆ together with the adjacent nitrogen atom each form a substituted or unsubstituted heterocyclic ring or a substituted or unsubstituted fused aromatic heterocyclic ring; or
   R₅ and R₆ each represent a substituted or unsubstituted cycloalkylideneamino group or a substituted or unsubstituted aromatic ring-fused cycloalkylidene group.
[A8] The composition according to any one of [A1] to [A7], wherein the compound that enhances phosphorylation of AMPK is a compound represented by General Formula (III) below or a prodrug thereof, or a pharmaceutically acceptable salt thereof 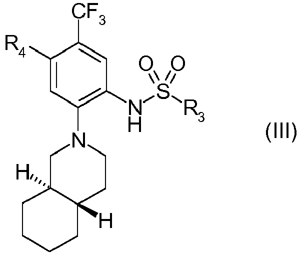 where R₃ represents a substituted or unsubstituted C₁₋₆ alkyl group, substituted or unsubstituted C₂₋₆ alkenyl group, substituted or unsubstituted C₆₋₁₀ aryl group, substituted or unsubstituted nitrogen-containing heterocyclic ring, or substituted or unsubstituted fused aromatic heterocyclic ring, and R₄ represents a hydrogen atom or a halogen atom.
[A9] The composition according to any one of [A1] to [A8], wherein the compound that enhances phosphorylation of AMPK is selected from the group consisting of 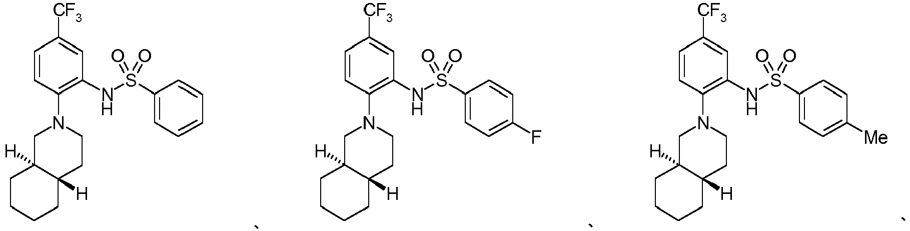 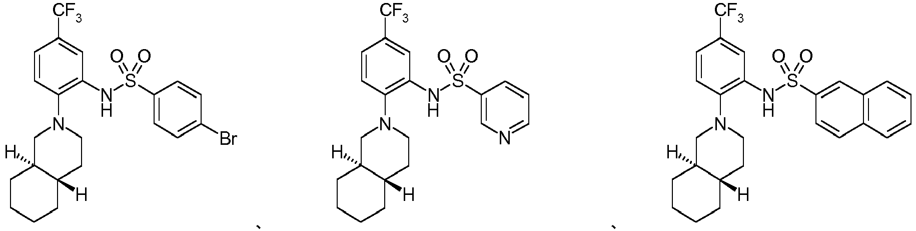 and combinations thereof.

### [Use]

In another aspect, the present disclosure relates to use of a compound that enhances phosphorylation of AMPK in a preservation solution, perfusate, or reperfusate for a human or animal organ, tissue, or part thereof before, during, or after transplant thereof, or an additive therefor. In one or more embodiments, the use of the present aspect is use of the composition according to the present disclosure as a preservation solution, perfusate, or reperfusate for a human or animal organ, tissue, or part thereof before, during, or after transplant thereof, or an additive therefor. The use of the composition containing the compound that enhances phosphorylation of AMPK as a preservation solution, perfusate, or additive can inhibit injury (cell death), which can be induced by ischemia, of a human or animal organ, tissue, or part thereof or can prolong the ischemic interval thereof in one or more non-limiting embodiments, or can inhibit injury (cell death), which can be induced by ischemia, of a human or animal organ, tissue, or part thereof or can inhibit or avoid ischemia-reperfusion injury in one or more non-limiting embodiments.

Accordingly, the present disclosure may further relate to one or more of the following embodiments.
[B1] Use of a compound that enhances phosphorylation of AMPK in a preservation solution, perfusate, or reperfusate for a human or animal organ, tissue, or part thereof before, during, or after transplant thereof, or an additive therefor.
[B2] The use according to [B1], wherein the use includes use of a composition containing the compound that enhances phosphorylation of AMPK, as a preservation solution, perfusate, or additive.
[B3] The use according to [B1] or [B2], wherein the use includes dissolving or mixing the compound that enhances phosphorylation of AMPK in a base solution to obtain an organ preservation solution or an organ perfusate, and the base solution is selected from the group consisting of UW solution, Bretschneider solution, Bretschneider's HTK solution, Euro-Collins solution, St. Thomas' Hospital solution, ET-Kyoto solution, plasma, serum, blood, and combinations thereof.
[B4] The use according to any one of [B1] to [B3], wherein in order to modify an organ preservation solution or an organ perfusate, the compound that enhances phosphorylation of AMPK is added to the organ preservation solution or the organ perfusate.
[B5] The use according to [B4], wherein the organ preservation solution or the organ perfusate is selected from the group consisting of UW solution, Bretschneider solution, Bretschneider's HTK solution, Euro-Collins solution, St. Thomas' Hospital solution, ET-Kyoto solution, plasma, serum, blood, and combinations thereof.
[B6] The use according to any one of [B1] to [B5], wherein the compound that enhances phosphorylation ofAMPK is a compound defined by any one of [A7] to [A9] or a prodrug thereof, or a pharmaceutically acceptable salt thereof.

### [Use in production]

In another aspect, the present disclosure relates to use of a compound that enhances phosphorylation of AMPK in production of a perfusate or preservation solution for a human or animal organ, tissue, or part thereof,
for inhibiting injury during preservation, storage, or transportation of an ex vivo organ or tissue,
for inhibiting reperfusion injury, for inhibiting graft ischemic injury,
for improving functional recovery after transplant of an organ or tissue, or
for inhibiting transplant failure.

In one or more embodiments, examples of the use of the present aspect include adding the compound that enhances phosphorylation of AMPK in such a manner that the concentration of said compound in the preservation solution or perfusate containing the compound added thereto is a concentration that can enhance the phosphorylation of AMPK, or 2 to 150 µM.

Accordingly, the present disclosure may further relate to one or more of the following embodiments.
[C1] Use of a compound that enhances phosphorylation ofAMPK in production of a perfusate or preservation solution for a human or animal organ, tissue, or part thereof, for inhibiting injury during preservation, storage, or transportation of an ex vivo organ or tissue,
   for inhibiting reperfusion injury, for inhibiting graft ischemic injury,
   for improving functional recovery after transplant of an organ or tissue, or
   for inhibiting transplant failure.
[C2] The use according to [C1], wherein the production includes dissolving or mixing the compound that enhances phosphorylation of AMPK in a base solution to obtain an organ preservation solution or an organ perfusate.
[C3] The use according to [C2], wherein the base solution is selected from the group consisting of UW solution, Bretschneider solution, Bretschneider's HTK solution, Euro-Collins solution, St. Thomas' Hospital solution, ET-Kyoto solution, plasma, serum, blood, and combinations thereof.
[C4] The use according to any one of [C1] to [C3], wherein the compound that enhances phosphorylation ofAMPK is a compound defined by any one of [A7] to [A9] or a prodrug thereof, or a pharmaceutically acceptable salt thereof.

### [Organ preservation method]

In another aspect, the present disclosure relates to a method of preserving, storing, or transporting an ex vivo human or animal organ, tissue, or part thereof, or a method for inhibiting injury during preservation, storage, or transportation thereof, the method including contacting the organ, tissue, or part thereof with a compound that enhances phosphorylation ofAMPK. In one or more embodiments, "contacting the organ, tissue, or part thereof with a compound that enhances phosphorylation of AMPK" in the method of the present aspect denotes contacting the organ, tissue, or part thereof with the composition according to the present disclosure, or contacting the organ, tissue, or part thereof with the medical composition according to the present disclosure, or contacting the organ, tissue, or part thereof with the preservation solution according to the present disclosure. In one or more embodiments, the contacting can be carried out by perfusion, dipping, rinsing, injection, or any combination thereof.

Accordingly, the present disclosure may further relate to one or more of the following embodiments.
[D1] A method of preserving, storing, or transporting an ex vivo human or animal organ, tissue, or part thereof, or a method for inhibiting injury during preservation, storage, or transportation thereof,
   the method including contacting the organ, tissue, or part thereof with a compound that enhances phosphorylation of AMPK.
[D2] The method according to [D1], wherein the method includes contacting a preservation solution containing the compound that enhances phosphorylation of AMPK with the organ, tissue, or part thereof.
[D3] The composition according to [D2], wherein the preservation solution is an organ preservation solution in which the compound that enhances phosphorylation of AMPK is dissolved or mixed in a base solution, and the base solution is selected from the group consisting of UW solution, Bretschneider solution, Bretschneider's HTK solution, Euro-Collins solution, St. Thomas' Hospital solution, ET-Kyoto solution, plasma, serum, blood, and combinations thereof.
[D4] The method according to [D3], wherein the contacting is selected from the group consisting of perfusion, dipping, rinsing, injection, and combinations thereof.
[D5] The method according to any one of [D1] to [D4], wherein the compound that enhances phosphorylation of AMPK is a compound defined by any one of [A7] to [A9] or a prodrug thereof, or a pharmaceutically acceptable salt thereof.

### [Transplant method]

In another aspect, the present disclosure relates to a method of transplanting an ex vivo human or animal organ, tissue, or part thereof, the method including contacting the organ, tissue, or part thereof with a compound that enhances phosphorylation of AMPK before, during, or after transplant thereof. In one or more embodiments, "contacting the organ, tissue, or part thereof with a compound that enhances phosphorylation of AMPK" in the method of the present aspect denotes contacting the organ, tissue, or part thereof with the composition according to the present disclosure, or contacting the organ, tissue, or part thereof with the medical composition or pharmaceutical composition according to the present disclosure, or contacting the organ, tissue, or part thereof with the preservation solution or perfusate according to the present disclosure. In one or more embodiments, the contacting can be carried out by perfusion, dipping, rinsing, injection, or any combination thereof.

Accordingly, the present disclosure may further relate to one or more of the following embodiments.
[E1] A method of transplanting an ex vivo human or animal organ, tissue, or part thereof, the method including contacting the organ, tissue, or part thereof with a compound that enhances phosphorylation of AMPK before, during, or after transplant thereof.
[E2] The method according to [E1], wherein the method includes contacting a preservation solution or perfusate containing the compound that enhances phosphorylation of AMPK with the organ, tissue, or part thereof.
[E3] The method according to [E2], wherein the preservation solution or perfusate is an organ preservation solution or an organ perfusate in which the compound that enhances phosphorylation of AMPK is dissolved or mixed in a base solution, and the base solution is selected from the group consisting of UW solution, Bretschneider solution, Bretschneider's HTK solution, Euro-Collins solution, St. Thomas' Hospital solution, ET-Kyoto solution, plasma, serum, blood, and combinations thereof.
[E4] The method according to [E3], wherein the contacting is selected from the group consisting of perfusion, dipping, rinsing, injection, and combinations thereof.
[E5] The method according to any one of [E1] to [E4], wherein the compound that enhances phosphorylation of AMPK is a compound defined by any one of [A7] to [A9] or a prodrug thereof, or a pharmaceutically acceptable salt thereof.

In the present disclosure, the organ is heart, lung, liver, kidney, pancreas, spleen, intestine, or bladder in one or more non-limiting embodiments, and examples of the part thereof include heart valve, blood vessel section, liver lobe, intestinal section, muscle preparation, and limb in one or more non-limiting embodiments. Furthermore, in the present disclosure, examples of the tissue or part thereof include corneas, skin tissues, and skin grafts in one or more non-limiting embodiments.

### [Treatment Method]

In another aspect, the present disclosure relates to a method of treating a disease caused by infarction, the method including using, as a reperfusate, a perfusate (blood) containing a compound that enhances phosphorylation of AMPK. In one or more embodiments, for example, the compound that enhances phosphorylation of AMPK in the perfusate is added in such a manner that the concentration of said compound is a concentration that can enhance the phosphorylation of AMPK or 2 to 150 µM. In one or more embodiments, the present aspect includes using, as a perfusate, blood or serum in which a compound that enhances phosphorylation of AMPK is dissolved, or using, as an additive to be administered to blood, a compound that enhances phosphorylation ofAMPK.

Accordingly, the present disclosure may further relate to one or more of the following embodiments.
[F1] A method of treating a disease caused by infarction, the method including carrying out reperfusion using a reperfusate containing a compound that enhances phosphorylation ofAMPK.
[F2] The method according to [F1], wherein the reperfusate is a reperfusate in which the compound that enhances phosphorylation of AMPK is dissolved or mixed in a base solution, and the base solution is selected from the group consisting of UW solution, Bretschneider solution, Bretschneider's HTK solution, Euro-Collins solution, St. Thomas' Hospital solution, ET-Kyoto solution, plasma, serum, blood, and combinations thereof.
[F3] The method according to [F1] or [F2], wherein the reperfusate is blood or serum in which the compound that enhances phosphorylation of AMPK is dissolved.
[F4] The method according to [F1] to [F3], wherein the method includes administering, to a subject, a composition containing the compound that enhances phosphorylation of AMPK before reperfusion or during reperfusion.
[F5] The method according to any one of [F1] to [F4], wherein the compound that enhances phosphorylation of AMPK is a compound defined by any one of [A7] to [A9] or a prodrug thereof, or a pharmaceutically acceptable salt thereof.

### [Pharmaceutical composition for cancer]

It was found that the compound that enhances phosphorylation of AMPK according to the present disclosure can induce cell death in cancer cells. Accordingly, in an aspect, the present disclosure relates to a pharmaceutical composition for cancer, the composition containing, as an active component, a compound that enhances phosphorylation ofAMPK.

The pharmaceutical composition of the present aspect can induce cell death in cancer cells. In the present disclosure, the expression "induce cell death in cancer cells" denotes reducing the survival rate of cancer cells and/or decreasing the number of cancer cells in one or more embodiments. In one or more non-limiting embodiments, examples of the cancer cells include brain tumor, glioblastoma, pancreatic ductal carcinoma, rhabdomyosarcoma, lung cancer, colon cancer, skin cancer, prostate cancer, breast cancer, and ovarian cancer.

In one or more embodiments, as described above, the pharmaceutical composition of the present aspect can prolong the survival of normal cells or non-cancer cells or inhibit cell death of normal cells or non-cancer cells.

Examples of the active component of the pharmaceutical composition of the present aspect include the above-mentioned compound that enhances phosphorylation of AMPK and include the above-mentioned compound represented by General Formula (I) or a prodrug thereof, or a pharmaceutically acceptable salt thereof in one or more embodiments.

In one or more embodiments of the present aspect, the enhancement of phosphorylation of AMPK is the enhancement of phosphorylation of AMPK mediated by a G protein-coupled receptor (GPCR).

In one or more embodiments, the cell death induced by the pharmaceutical composition of the present aspect is programmed cell death. In the present disclosure, the programmed cell death include apoptosis, cell death with autophagy (hereinafter also referred to simply as "autophagic cell death"), and necrotic programmed cell death. In one or more embodiments, the cell death induced by the pharmaceutical composition of the present aspect is autophagic cell death. In one or more embodiments, the autophagic cell death can be confirmed by enhanced expression of an autophagy marker (for example, LC3-II).

In one or more embodiments, the pharmaceutical composition of the present aspect can be formed in a suitable dosage form for the type of administration by applying a well-known formulation technique. Examples of the type of administration include, but are not limited to, oral administration in the dosage form of tablets, capsules, granules, powders, pills, lozenges, syrups, liquids, etc. Alternatively, examples thereof may include parenteral administration in the dosage form of injections, liquids, aerosols, suppositories, patches, cataplasms, lotions, liniments, ointments, eye drops, etc. These formulations can be produced by well-known methods using additives such as, but not limited to, excipients, lubricants, binders, disintegrants, stabilizers, corrigents, diluents, etc.

Examples of the excipients may include, but are not limited to, starches such as starch, potato starch, and corn starch, lactose, crystalline cellulose, and calcium hydrogen phosphate. Examples of the coating agents may include, but are not limited to, ethyl cellulose, hydroxypropyl cellulose, hydroxypropyl methylcellulose, shellac, talc, carnauba wax, and paraffin. Examples of the binders may include, but are not limited to, polyvinylpyrrolidone, macrogol, and the same compounds as those mentioned as the excipients. Examples of the disintegrants may include, but are not limited to, the same compounds as those mentioned as the excipients as well as chemically modified starches and celluloses such as croscarmellose sodium, sodium carboxymethyl starch, and cross-linked polyvinylpyrrolidone. Examples of the stabilizers may include, but are not limited to, parahydroxybenzoic acid esters such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid. Examples of the corrigents may include, but are not limited to, commonly used sweeteners, acidulants, and flavors.

In the production of the liquids, the solvent that can be used is, but is not limited to, ethanol, phenol, chlorocresol, purified water, or distilled water, and for example, a surfactant or an emulsifier also can be used if necessary. Examples of the surfactant or emulsifier may include, but are not limited to, polysorbate 80, polyoxyl 40 stearate, and lauromacrogol.

The method of using the pharmaceutical composition of the present aspect may vary depending on, for example, the symptoms, age, and administration method. The method of using the pharmaceutical composition may be, but is not limited to, a method of intermittently or continuously administering it orally, transdermally, submucosally, subcutaneously, intramuscularly, intravascularly, intracerebrally, or intraperitoneally in such a manner that the concentration of the compound represented by General Formula (I), which is an active component, in the body is between 100 nM and 1 mM. In a non-limiting embodiment, in the case of oral administration, for example, the pharmaceutical composition may be administered to a subject (an adult in the case of a human) in a dosage of 0.01mg (preferably 0.1 mg) as a lower limit to 2000 mg (preferably 500 mg and more preferably 100 mg) as an upper limit, in terms of the compound represented by General Formula (I), once or a few times a day depending on the symptoms. In a non-limiting embodiment, in the case of intravenous administration, for example, the pharmaceutical composition may be administered to a subject (an adult in the case of a human) in a dosage of 0.001mg (preferably 0.01 mg) as a lower limit to 500 mg (preferably 50 mg) as an upper limit once or a few times a day depending on the symptom.

Accordingly, the present disclosure may further relate to one or more of the following embodiments.
[G1] A pharmaceutical composition for inducing programmed cell death in cancer cells, the pharmaceutical composition containing, as an active component, a compound that enhances phosphorylation of AMPK.
[G2] A pharmaceutical composition for inducing cell death in cancer cells to prolong the survival of normal cells, the pharmaceutical composition containing, as an active component, a compound that enhances phosphorylation of AMPK.
[G3] The pharmaceutical composition according to [G1] or [G2], wherein the enhancement of phosphorylation of AMPK is the enhancement of phosphorylation of AMPK mediated by GPCR.
[G4] The pharmaceutical composition according to any one of [G1] to [G3], wherein the active component is a compound that enhances phosphorylation of AMPK mediated by GPCR to induce autophagic cell death in cancer cells.
[G5] The pharmaceutical composition according to any one of [G1] to [G4], wherein the compound that enhances phosphorylation of AMPK is a compound defined by any one of [A7] to [A9] or a prodrug thereof, or a pharmaceutically acceptable salt thereof.
[G6] A pharmaceutical composition, containing, as an active component, a compound defined by any one of [A7] to [A9] or a prodrug thereof, or a pharmaceutically acceptable salt thereof.
[G7] The pharmaceutical composition according to [G6], for cancer prevention, amelioration, progression inhibition, and/or treatment.
[G8] Use of a compound that enhances phosphorylation of AMPK, in production of a pharmaceutical composition according to any one of [G1] to [G7].

### [Method of cancer prevention, amelioration, progression inhibition, and/or treatment]

As described above, the use of the pharmaceutical composition of the present aspect allows a method of cancer prevention, amelioration, progression inhibition, and/or treatment to be carried out.

Accordingly, the present disclosure may further relate to one or more of the following embodiments.

[H1] A method of cancer prevention, amelioration, progression inhibition, and/or treatment, the method including administering a pharmaceutical composition according to any one of [G1] to [G7] to a subject.

### [Method of inducing programmed cell death in cancer cells]

It was found that the compound that enhances phosphorylation of AMPK according to the present disclosure was able to induce programmed cell death in cancer cells. Accordingly, in an aspect, the present disclosure relates to a method of inducing programmed cell death in cancer cells by using a compound that enhances phosphorylation ofAMPK. In the method according to the present aspect, the programmed cell death is autophagic cell death in one or more embodiments. In one or more embodiments, the autophagic cell death can be confirmed by enhanced expression of an autophagy marker (for example, LC3-II). In one or more embodiments of the present aspect, the enhancement of phosphorylation of AMPK is the enhancement of phosphorylation of AMPK mediated by a G protein-coupled receptor (GPCR). The method according to the present aspect can be carried out in vivo, in vitro, or ex vivo.

Accordingly, the present disclosure may further relate to one or more of the following embodiments.
[I1] A method of inducing programmed cell death in cancer cells, the method including contacting the cancer cells with a compound that enhances phosphorylation of AMPK or administering, to a biological body with the cancer cells, a compound that enhances phosphorylation ofAMPK.
[I2] A method of inducing programmed cell death in cancer cells, the method including contacting cancer cells with a compound defined by any one of [A7] to [A9] or a prodrug thereof, or a pharmaceutically acceptable salt thereof, or administering a compound defined by any one of [A7] to [A9] or a prodrug thereof, or a pharmaceutically acceptable salt thereof to a biological body with cancer cells.
[I3] The method according to [I1] or [I2], wherein the programmed cell death is autophagic cell death.

### [Examples]

Hereinafter, the present disclosure will be described in more detail with reference to examples, but these examples are illustrative, and the present disclosure is not limited to these examples. It should be noted that the entirety of the documents cited in the present disclosure is incorporated as a part of the present disclosure.

### Production Example 1: Production of Compound 1

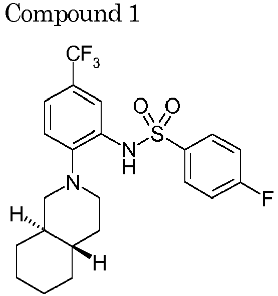

Compound 1 was synthesized as follows:

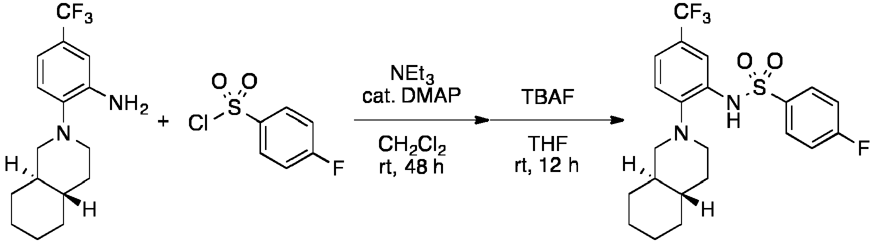

To a dichloromethane (20 mL) solution of 2-[(4aS,8aR)-octahydroisoquinolin-2(1H)-yl]-5-(trifluoromethyl)aniline (1.20 g, 4.03 mmol) synthesized by the method described in the document (PCT Int. Appl. (2009), WO2009119167 A120091001.), 4-fluorobenzenesulfonyl chloride (2.90 g, 14.9 mmol, commercial product), triethylamine (1.00 mL, 7.23 mmol, commercial product), and N,N-dimethyl-4-aminopyridine (10 mg, 0.082 mmol, commercial product) were added sequentially at room temperature and thereafter this was stirred for 48 hours. Water was added to this reaction mixture to stop the reaction, followed by extraction with ethyl acetate (×3). This was washed with saturated saline and then dried over anhydrous sodium sulfate. After this was filtered, the filtrate was concentrated under reduced pressure. Next, to the tetrahydrofuran (20 mL) solution which was the resultant reaction crude product, tetra-n-butylammonium fluoride (1.0 M in THF, 10 mL, 10 mmol) was added at room temperature, and this was stirred for 12 hours. After this was concentrated under reduced pressure, the resultant reaction crude product was purified by silica gel column chromatography (Biotage, SNAP Ultra 100 g, hexane/ethyl acetate = 50/1 to 20/1) and then was recrystallized (ethyl acetate/hexane). Thus, 4-fluoro-N-(2-((4aS,8aR)-octahydroisoquinolin-2(1H)-yl) -5-(trifluoromethyl)phenyl)benzenesulfonamide (647 mg, 1.42 mmol, 35.2%) (Compound 1) was obtained as colorless crystals.

TLC Rf 0.44 (hexane/ethyl acetate = 10/1); ¹H NMR (CDCl₃, 400 MHz) δ 0.89-1.13 (m, 3H), 1.22-1.48 (m, 5H), 1.50-1.72 (m, 2H), 1.73-1.81 (m, 2H), 2.24 (dd, 1H, J =10.8, 10.8 Hz), 2.37-2.42 (m, 1H), 2.50-2.62 (m, 2H), 7.09-7.16 (m, 3H), 7.26-7.30 (m, 1H), 7.82-7.88 (m, 3H), 7.98 (br s, 1H).

### Production Example 2: Production of Compound 2

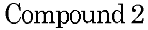

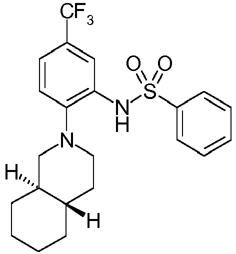

Compound 2 was synthesized as follows:

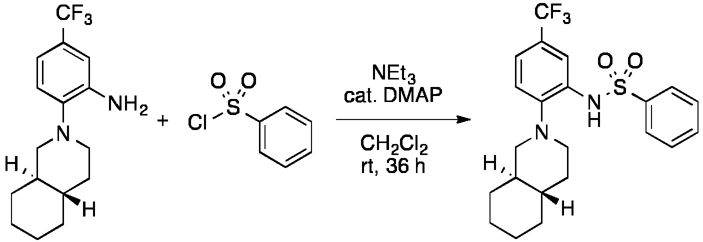

To a dichloromethane (10 mL) solution of 2-[(4aS,8aR)-octahydroisoquinolin-2(1H)-yl]-5-(trifluoromethyl)aniline (120 mg, 0.403 mmol) synthesized by the method described in the document (PCT Int. Appl. (2009), WO2009119167 A120091001.), benzenesulfonyl chloride (77 µL, 0.60 mmol, commercial product), triethylamine (0.10 mL, 0.72 mmol, commercial product), and N,N-dimethyl-4-aminopyridine (10 mg, 0.082 mmol, commercial product) were added sequentially at room temperature and thereafter this was stirred for 36 hours. Water was added to this reaction mixture to stop the reaction, followed by extraction with ethyl acetate (×3). This was washed with saturated saline and then dried over anhydrous sodium sulfate. After this was filtered, the filtrate was concentrated under reduced pressure. The resultant reaction crude product was purified by silica gel column chromatography (Kanto Chemical Co., Inc., neutral and spherical, 10 g, hexane/ethyl acetate = 20/1) and then was recrystallized (ethyl acetate/hexane). Thus, N-(2-((4aS,8aR)-octahydroisoquinolin-2(1H)-yl)-5-(trifluoromethyl)phenyl)benzenesulfo namide (146 mg, 0.33 mmol, 83.1%) (Compound 2) was obtained as colorless crystals.

TLC Rf 0.19 (hexane/ethyl acetate = 20/1); ¹H NMR (CDCl₃, 400 MHz) δ 0.78-1.12 (m, 3H), 1.13-1.48 (m, 5H), 1.55-1.80 (m, 4H), 2.20 (dd, 1H, J =10.4, 10.4 Hz), 2.31-2.38 (m, 1H), 2.49-2.59 (m, 2H), 7.12 (d, 1H, J = 8.4 Hz), 7.26 (dd, 1H, J = 8.4, 1.6 Hz), 7.42-7.47 (m, 2H), 7.51-7.57 (m, 1H), 7.81-7.85 (m, 2H), 7.87 (dd, 1H, J = 1.6 Hz), 7.97 (br s, 1H).

### Production Example 3: Production of Compound 3

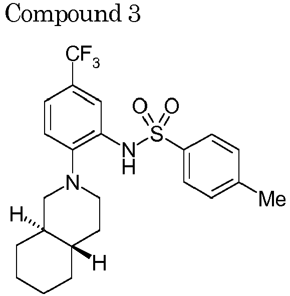

Compound 3 was synthesized as follows:

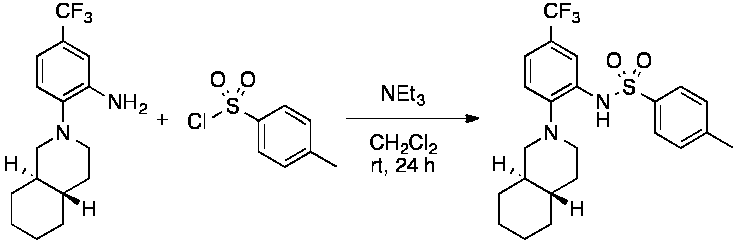

To a dichloromethane (5.0 mL) solution of 2-[(4aS,8aR)-octahydroisoquinolin-2(1H)-yl]-5-(trifluoromethyl)aniline (600 mg, 2.03 mmol) synthesized by the method described in the document (PCT Int. Appl. (2009), WO2009119167 A1 20091001.), 4-toluenesulfonyl chloride (460 mg, 2.41 mmol, commercial product) and triethylamine (0.33 mL, 2.39 mmol, commercial product) were added sequentially at room temperature and thereafter this was stirred for 24 hours. Water was added to this reaction mixture to stop the reaction, followed by extraction with ethyl acetate (×3). This was washed with saturated saline and then dried over anhydrous sodium sulfate. After this was filtered, the filtrate was concentrated under reduced pressure. The resultant reaction crude product was purified by silica gel column chromatography (Kanto Chemical Co., Inc., neutral and spherical, 20 g, hexane/ethyl acetate = 50/1 to 20/1) and then was recrystallized (ethyl acetate/hexane). Thus, N-(2-((4aS,8aR)-octahydroisoquinolin-2(1H)-yl) -5-(trifluoromethyl)phenyl)-4-toluenesulfonamide (180 mg, 0.397 mmol, 19.9%) (Compound 3) was obtained as colorless crystals.

TLC Rf 0.43 (hexane/ethyl acetate = 10/1); ¹H NMR (CDCl₃, 400 MHz) δ 0.88-1.14 (m, 3H), 1.24-1.48 (m, 5H), 1.50-1.82 (m, 4H), 2.20 (dd, 1H, J =10.8, 10.8 Hz), 2.35-2.41 (m, 4H), 2.54-2.59 (m, 2H), 7.12 (d, 1H, J = 8.0 Hz), 7.22-7.26 (m, 3H), 7.70-7.73 (AA'BB', 2H), 7.86 (d, 1H, J = 2.0 Hz), 7.95 (br s, 1H).

### Production Example 4: Production of Compound 4

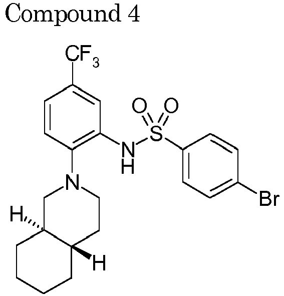

Compound 4 was synthesized as follows:

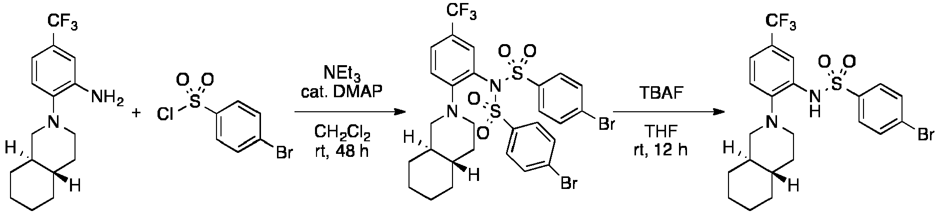

To a dichloromethane (5.0 mL) solution of 2-[(4aS,8aR)-octahydroisoquinolin-2(1H)-yl]-5-(trifluoromethyl)aniline (120 mg, 0.403 mmol) synthesized by the method described in the document (PCT Int. Appl. (2009), WO2009119167 A1 20091001.), 4-bromobenzenesulfonyl chloride (310 mg, 1.21 mmol, commercial product), triethylamine (0.20 mL, 1.45 mmol, commercial product), and N,N-dimethyl-4-aminopyridine (10 mg, 0.082 mmol, commercial product) were added sequentially at room temperature and thereafter this was stirred for 48 hours. Water was added to this reaction mixture to stop the reaction, followed by extraction with ethyl acetate (×3). This was washed with saturated saline and then dried over anhydrous sodium sulfate. After this was filtered, the filtrate was concentrated under reduced pressure. The resultant reaction crude product was reprecipitated (ethyl acetate/hexane) and thereby a bissulfonylimide intermediate (232 mg, 0.315 mmol, 78.4%) was obtained.

Next, to a tetrahydrofuran (20 mL) solution which was the resultant bissulfonylimide intermediate (152 mg, 0.207 mmol), tetra-n-butylammonium fluoride (1.0 M in THF, 1.0 mL, 1.0 mmol) was added at room temperature and then this was stirred for 12 hours. After this was concentrated under reduced pressure, the resultant reaction crude product was purified by silica gel column chromatography (Kanto Chemical Co., Inc., neutral and spherical, 10 g, hexane/ethyl acetate = 20/1 to 10/1) and then was recrystallized (ethyl acetate/hexane). Thus, 4-bromo-N-(2-((4aS,8aR)-octahydroisoquinolin-2(1H)-yl)-5-(trifluoromethyl)phenyl)ben zenesulfonamide (101 mg, 0.195 mmol, 94.4%) (Compound 4) was obtained as colorless crystals.

TLC Rf 0.23 (hexane/ethyl acetate = 20/1); ¹H NMR (CDCl₃, 400 MHz) δ 0.88-1.12 (m, 3H), 1.32-1.50 (m, 5H), 1.61-1.73 (m, 2H), 1.73-1.81 (m, 2H), 2.23 (dd, 1H, J =10.8, 10.8 Hz), 2.32-2.40 (m, 1H), 2.50-2.62 (m, 2H), 7.15 (d, 1H, J = 8.4 Hz), 7.28 (dd, 1H, J = 8.4, 1.6 Hz), 7.56-7.61 (AA'BB', 2H), 7.67-7.70 (AA'BB', 2H), 7.84 (d, 1H, J = 1.6 Hz), 7.99 (br s, 1H).

### Production Example 5: Production of Compound 5

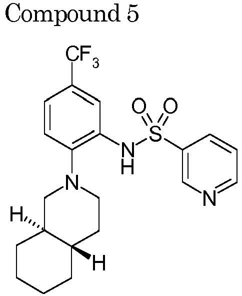

Compound 5 was synthesized as follows:

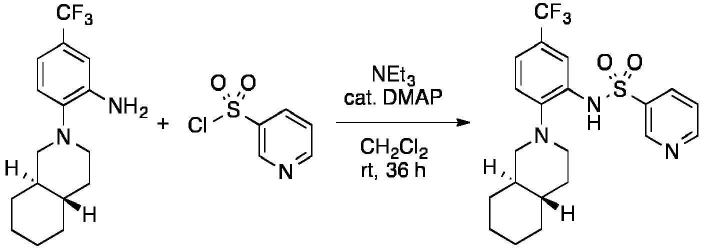

To a dichloromethane (5.0 mL) solution of 2-[(4aS,8aR)-octahydroisoquinolin-2(1H)-yl]-5-(trifluoromethyl)aniline (120 mg, 0.402 mmol) synthesized by the method described in the document (PCT Int. Appl. (2009), WO2009119167 A1 20091001.), 3-pyridinesulfonyl chloride hydrochloride (130 mg, 0.607 mmol, commercial product), triethylamine (0.40 mL, 2.89 mmol, commercial product), and N,N-dimethyl-4-aminopyridine (10 mg, 0.082 mmol, commercial product) were added sequentially at room temperature and thereafter this was stirred for 48 hours. Water was added to this reaction mixture to stop the reaction, followed by extraction with ethyl acetate (×3). This was washed with saturated saline and then dried over anhydrous sodium sulfate. After this was filtered, the filtrate was concentrated under reduced pressure. The resultant reaction crude product was purified by silica gel column chromatography (Kanto Chemical Co., Inc., neutral and spherical, 10 g, hexane/ethyl acetate = 5/1 to 1/1) and then was purified again by preparative TLC (hexane/ethyl acetate =1/1). Thus, N-(2-((4aS,8aR)-octahydroisoquinolin-2(1H)-yl)-5-(trifluoromethyl)phenyl)-3-pyridinesulfonamide (22.8 mg, 0.0519 mmol, 12.9%) (Compound 5) was obtained as colorless crystals.

TLC Rf 0.21 (hexane/ethyl acetate = 2/1); ¹H NMR (CDCl₃, 400 MHz) δ 0.87-1.13 (m, 3H), 1.22-1.51 (m, 5H), 1.52-1.81 (m, 4H), 2.24 (dd, 1H, J =10.810.8 Hz), 2.36-2.42 (m, 1H), 2.51-2.63 (m, 2H), 7.16 (d, 1H, J = 8.4 Hz), 7.30 (dd, 1H, J = 8.4,1.6 Hz), 7.41 (ddd, 1H, J = 8.0, 4.8, 0.8 Hz), 7.85 (d, 1H, J = 1.6 Hz), 8.06-8.13 (m, 2H), 8.76 (dd, 1H, J = 4.8, 1.2 Hz), 9.04 (dd, 1H, J = 2.4, 0.8 Hz).

### Production Example 6: Production of Compound 6

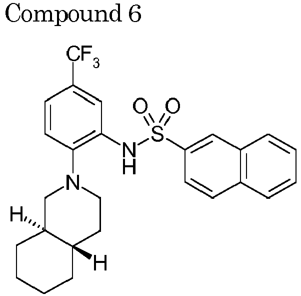

Compound 6 was synthesized as follows:

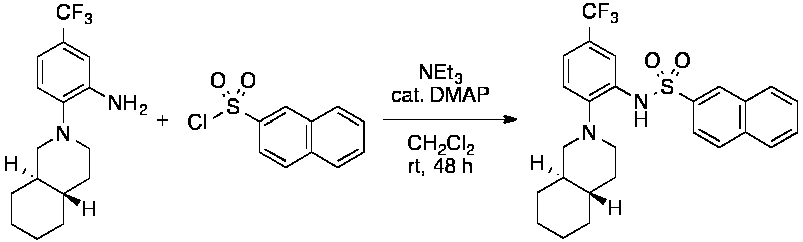

To a dichloromethane (5.0 mL) solution of 2-[(4aS,8aR)-octahydroisoquinolin-2(1H)-yl]-5-(trifluoromethyl)aniline (120 mg, 0.402 mmol) synthesized by the method described in the document (PCT Int. Appl. (2009), WO2009119167 A1 20091001.), 4-toluenesulfonyl chloride (140 mg, 0.617 mmol, commercial product), triethylamine (0.10 mL, 0.72 mmol, commercial product), and N,N-dimethyl-4-aminopyridine (10 mg, 0.082 mmol, commercial product) were added sequentially at room temperature and thereafter this was stirred for 48 hours. Water was added to this reaction mixture to stop the reaction, followed by extraction with ethyl acetate (×3). This was washed with saturated saline and then dried over anhydrous sodium sulfate. After this was filtered, the filtrate was concentrated under reduced pressure. The resultant reaction crude product was purified by silica gel column chromatography (Kanto Chemical Co., Inc., neutral and spherical, 20 g, hexane/ethyl acetate = 50/1 to 20/1) and then was recrystallized (ethyl acetate/hexane). Thus, N-(2-((4aS,8aR)-octahydroisoquinolin-2(1H)-yl)-5-(trifluoromethyl)phenyl)-2-naphthale nesulfonamide (50.6 mg, 0.103 mmol, 25.7%) (Compound 6) was obtained as colorless crystals.

TLC Rf 0.34 (hexane/ethyl acetate = 10/1); ¹H NMR (CDCl₃, 400 MHz) δ 0.82-1.12 (m, 3H), 1.23-1.80 (m, 9H), 2.14 (dd, 1H, J =11.2, 11.2 Hz), 2.24-2.30 (m, 1H), 2.51-2.56 (m, 2H), 7.08 (d, 1H, J = 8.4 Hz), 7.20-7.24 (m, 1H), 7.57-7.65 (m, 2H), 7.75 (dd, 1H, J = 8.6, 1.6 Hz), 7.85-7.93 (m, 3H), 7.97 (d, 1H, J = 2.0 Hz), 8.08 (br s, 1H), 8.45 (s, 1H).

### [Experimental Example 1: Enhancement of phosphorylation of AMPK by Compound 1]

Compound 1 was added to mouse fibroblast NIH/3T3 cells under serum-free conditions, the cells were harvested over a period of time to prepare a disrupted cell suspension, and phosphorylated AMPKα, total AMPKα, phosphorylated ACC, and total ACC were detected by Western blotting. ACC denotes acetyl-CoA carboxylase.

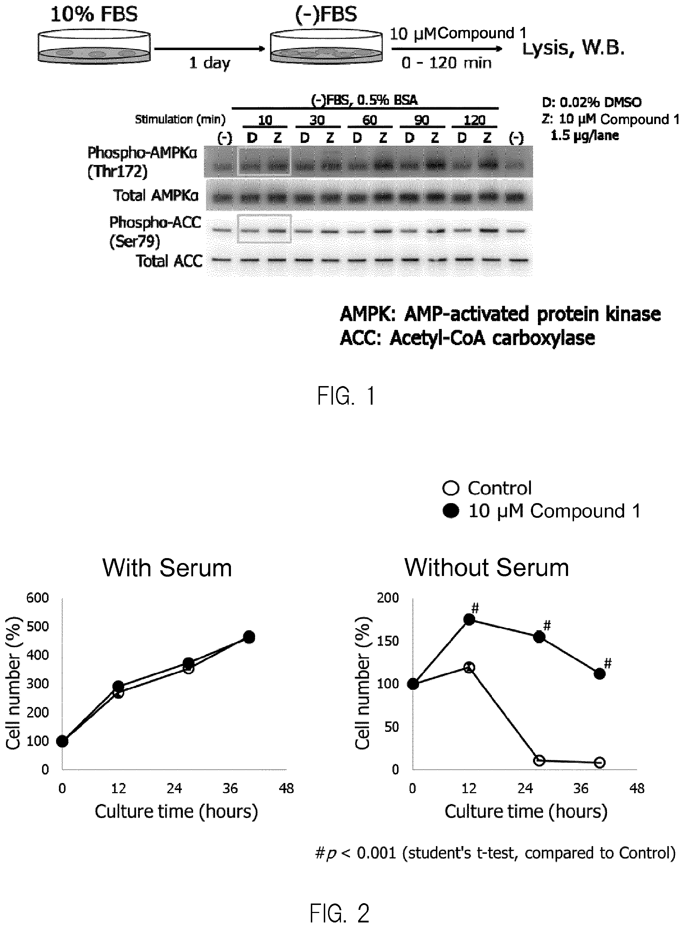

An example of the results is shown in Fig. 1. Fig. 1 shows an example of the results obtained by detecting, by Western blotting, the abundance of phosphorylated AMPKα, total AMPKα, phosphorylated ACC, and total ACC at 10, 30, 60, 90, and 120 minutes after the addition of Compound 1.

As shown in Fig. 1, phosphorylation of AMPK and ACC was enhanced within 10 minutes after the addition of Compound 1. Furthermore, phosphorylation of AMPK and ACC was maintained even 16 hours after the addition (data not shown).

### [Experimental Example 2: Inhibition of cell death by Compound 1]

NIH/3T3 cells were cultured under serum-containing or serum-free conditions, and after a predetermined period of time, a WST-8 assay was performed to confirm the cell number (N=6).

An example of the results is shown in Fig. 2. FIG. 2 is a graph comparing the viable cell numbers in the serum-starved condition in the presence and absence of Compound 1. As shown in Fig. 2, Compound 1 significantly inhibited cell death caused by serum starvation. Similarly with respect to Compounds 2, 3, 4, and 6, a cell death inhibitory effect equivalent to that of Compound 1 was observed. Furthermore, similarly with respect to the cell death that occurred when the cells were stored at 4°C for 24 hours and then rewarmed to 37°C, the cell death inhibitory effect was observed when Compound 1 was added. Moreover, similarly with respect to TNF-α induced apoptosis of mouse fibroblast MEF, that is, proinflammatory cell death, the cell death inhibitory effect was observed when Compound 1 was added.

### [Experimental Example 3: Inhibition of cell death in primary culture by Compound 1]

Mouse spinal cord macrophages were cultured under serum-containing or serum-free conditions, and after a predetermined period of time, a WST-8 assay was performed to confirm the cell number (N=6).

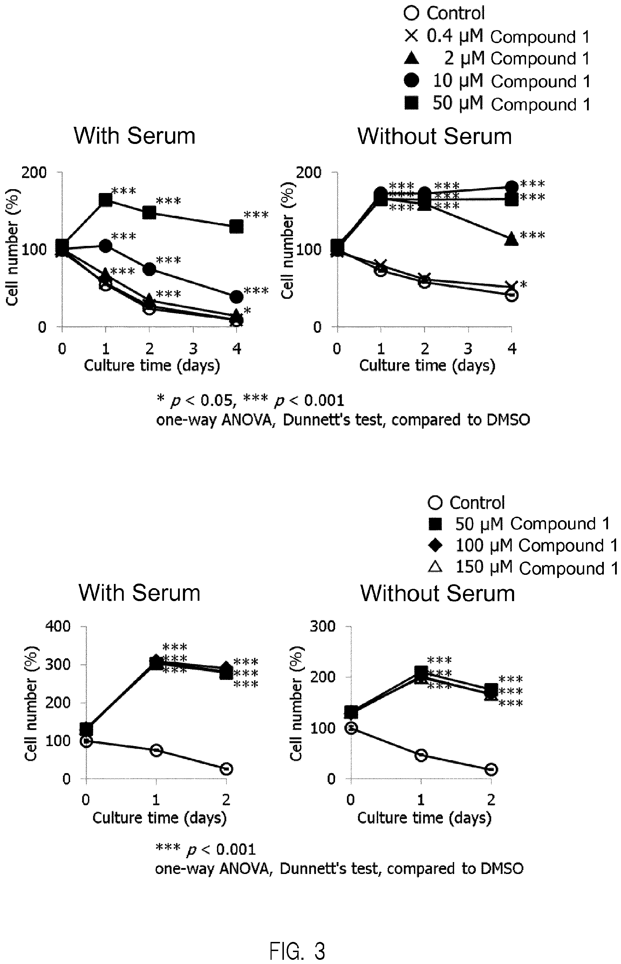

An example of the results is shown in Fig. 3. Fig. 3 shows graphs comparing the viable cell numbers of primary cultured cells in the serum-starved condition in the presence and absence of Compound 1. As shown in Fig. 3, Compound 1 significantly inhibited cell death in primary culture independent of serum starvation.

### [Experimental Example 4: Inhibition of hepatic injury in rat liver warm ischemia reperfusion injury model by Compound 1]

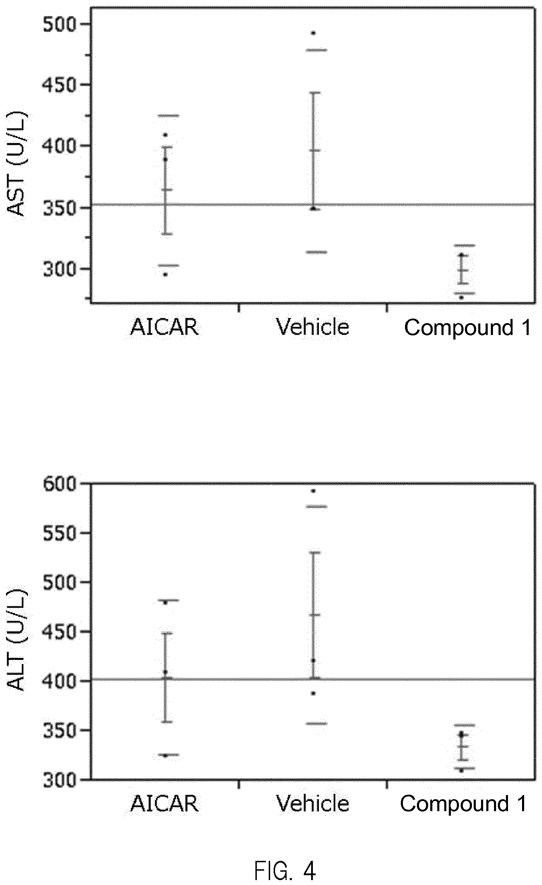

An experiment was performed with a rat liver warm ischemia reperfusion model, in which LEW rats of eight to nine weeks of age were subjected to laparotomy under general anesthesia by endotracheal intubation, and after 20 minutes of warm ischemia applied by blocking the hepatic portal region, the blocking was released and reperfusion was carried out. With respect to positive subjects, using AICAR (aminoimidazole-4-carboxamide rebonucleoside), which is an AMPK activator, a solvent, Compound 1 (0.3 mg/kg), and AICAR (100 mg/kg) were administered, respectively, before ischemia, and aspartate aminotransferase (AST) and alanine aminotransferase (ALT), which are liver deviation enzymes, in peripheral blood were measured six hours after the reperfusion (N=3). Pre-administering Compound 1 to a rat having a liver warm ischemia reperfusion injury inhibited the elevation of liver deviation enzymes (Fig. 4) to alleviate hepatic injury.

### [Experimental Example 5: Compound 1 induces cell death in cancer cells]

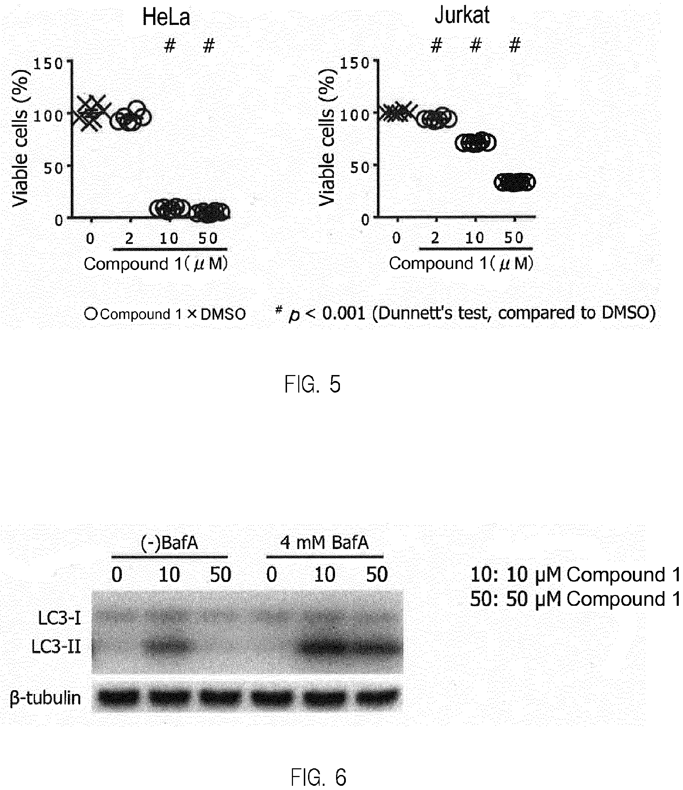

Compound 1 was able to induce cell death in HeLa cells which are human cervical cancer cell lines, Jurkat cells which are human T-cell leukemia lines, and SH-SY5Y cells which are human neuroblast lines. That is, Compound 1 was found to have an anticancer cell effect. An example of the results is shown in Fig. 5.

Fig. 5 shows examples of the results of an experiment in which Compound 1 was added to cultured HeLa and Jurkat cells at the concentrations indicated in Fig. 5, and three days later, a WST-8 assay was performed to confirm the numbers of the cells. As shown in Fig. 5, Compound 1 was confirmed to exhibit an anticancer effect.

### [Experimental Example 6: Compound 1 induces autophagy in cancer cells]

Bafilomycin A1 (BafA) is an autophagy inhibitor. BafA was added to HeLa cells for one hour and then Compound 1 was added thereto. After this was cultured for 21 hours, the cells were harvested to prepare a disrupted cell suspension, and the amount of LC3-II was confirmed by Western blotting. LC3-II is an autophagy marker. An example of the results is shown in Fig. 6. As shown in Fig. 6, in the presence of 4 mM of BafA, the addition of Compound 1 considerably increased the amount of LC3-II that was detected. From this result, it was confirmed that autophagy was induced in cancer cells by Compound 1.

### [Experimental Example 7: Compound 1 induces autophagic cell death in cancer cells]

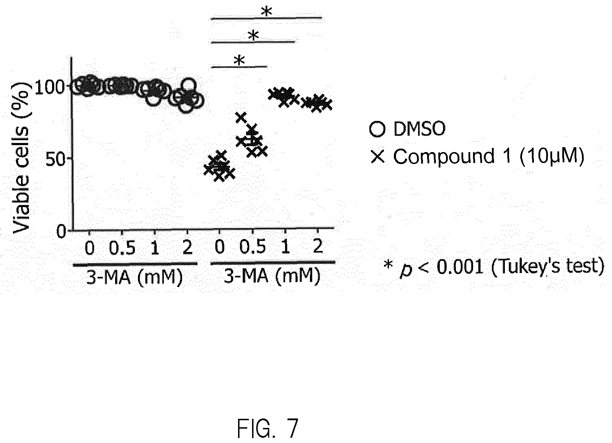

3-Methyladenine (3-MA) is a class III PI3K inhibitor and is also an autophagy inhibitor. 3-MA was added to HeLa cells for one hour, and three days later, a WST-8 assay was performed to confirm the number of the cells. An example of the results is shown in Fig. 7.

As shown in Fig. 7, the survival rate of the cells was improved depending on the amount of 3-MA that was added. Thus, it was confirmed that cell death induced by Compound 1 in cancer cells was autophagic.

## Claims

1. A composition that prolongs the survival of cells, the composition comprising, as an active component, a compound that enhances phosphorylation of AMP-activated protein kinase (AMPK).

2. The composition according to claim 1, wherein the composition is a medical composition for inhibiting injury or cell death during perfusion, reperfusion, or preservation of a human or animal organ, tissue, or part thereof.

3. The composition according to claim 1, wherein the composition is a pharmaceutical composition for inhibiting injury or cell death during perfusion or reperfusion of a human or animal organ, tissue, or part thereof.

4. The composition according to any one of claims 1 to 3, wherein the composition is an organ preservation solution or an organ perfusate in which the compound that enhances phosphorylation of AMPK is dissolved or mixed in a base solution, and the base solution is selected from the group consisting of UW solution, Bretschneider solution, Bretschneider's HTK solution, Euro-Collins solution, St. Thomas' Hospital solution, ET-Kyoto solution, plasma, serum, blood, and combinations thereof.

5. The composition according to any one of claims 1 to 3, wherein the composition is an additive for modifying an organ preservation solution or an organ perfusate.

6. The composition according to claim 5, wherein the organ preservation solution or the organ perfusate is selected from the group consisting of UW solution, Bretschneider solution, Bretschneider's HTK solution, Euro-Collins solution, St. Thomas' Hospital solution, ET-Kyoto solution, plasma, serum, blood, and combinations thereof.

7. The composition according to any one of claims 1 to 6, wherein the compound that enhances phosphorylation of AMPK is a compound represented by General Formula (I) below or a prodrug thereof, or a pharmaceutically acceptable salt thereof 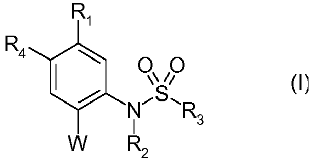
where R₁ represents a hydrogen atom, substituted or unsubstituted C₁₋₆ alkyl group, substituted or unsubstituted C₂₋₆ alkenyl group, substituted or unsubstituted C₂₋₆ alkynyl group, substituted or unsubstituted C₆₋₁₀ aryl group, halogen atom, nitro group, cyano group, azide group, hydroxy group, substituted or unsubstituted C₁₋₆ alkoxy group, substituted or unsubstituted C₁₋₆ alkylthio group, substituted or unsubstituted C₁₋₆ alkylsulfonyl group, carboxy group, formyl group, substituted or unsubstituted C₁₋₆ alkoxycarbonyl group, acyl group, acylamino group, or sulfamoyl group;
R₂ represents a hydrogen atom, substituted or unsubstituted C₁₋₆ alkyl group, or substituted or unsubstituted aryl group;
R₃ represents a substituted or unsubstituted C₁₋₆ alkyl group, substituted or unsubstituted C₂₋₆ alkenyl group, substituted or unsubstituted C₆₋₁₀ aryl group, substituted or unsubstituted nitrogen-containing heterocyclic ring, or substituted or unsubstituted fused aromatic heterocyclic ring;
R₄ represents a hydrogen atom or a halogen atom; and
W represents a hydrogen atom, substituted or unsubstituted C₁₋₆ alkyl group, substituted or unsubstituted C₆₋₁₀ aryl group, halogen atom, hydroxy group, substituted or unsubstituted C₁₋₆ alkoxy group, substituted or unsubstituted C₁₋₆ alkylthio group, substituted or unsubstituted nitrogen-containing heterocyclic ring, or substituted or unsubstituted fused aromatic heterocyclic ring, or W is represented by General Formula (II) below: 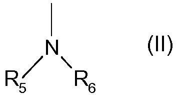
where R₅ and R₆, which are identical to or different from each other, each represent a hydrogen atom, substituted or unsubstituted C₁₋₆ alkyl group, substituted or unsubstituted nitrogen-containing heterocyclic ring, substituted or unsubstituted fused aromatic heterocyclic ring, acyl group, or acylamino group; or
R₅ and R₆ together with an adjacent nitrogen atom each form a substituted or unsubstituted heterocyclic ring or a substituted or unsubstituted fused aromatic heterocyclic ring; or
R₅ and R₆ each represent a substituted or unsubstituted cycloalkylideneamino group or a substituted or unsubstituted aromatic ring-fused cycloalkylidene group.

8. The composition according to any one of claims 1 to 7, wherein the compound that enhances phosphorylation of AMPK is a compound represented by General Formula (III) below or a prodrug thereof, or a pharmaceutically acceptable salt thereof 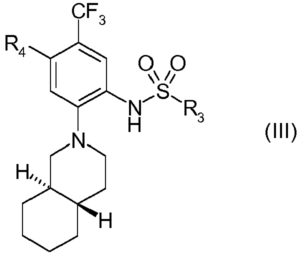 where R₃ represents a substituted or unsubstituted C₁₋₆ alkyl group, substituted or unsubstituted C₂₋₆ alkenyl group, substituted or unsubstituted C₆₋₁₀ aryl group, substituted or unsubstituted nitrogen-containing heterocyclic ring, or substituted or unsubstituted fused aromatic heterocyclic ring, and R₄ represents a hydrogen atom or a halogen atom.

9. The composition according to any one of claims 1 to 8, wherein the compound that enhances phosphorylation of AMPK is selected from the group consisting of 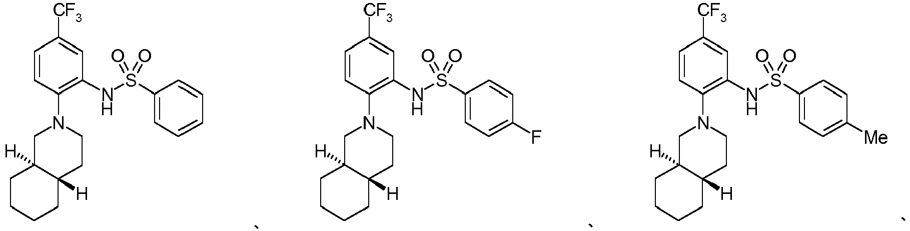 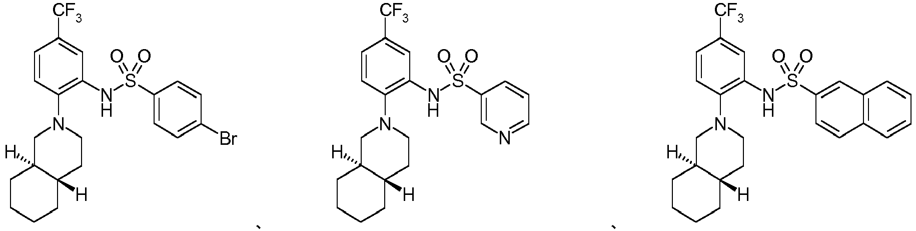 and combinations thereof.

10. Use of a compound that enhances phosphorylation of AMPK in a preservation solution, perfusate, or reperfusate for a human or animal organ, tissue, or part thereof before, during, or after transplant thereof, or an additive therefor.

11. Use of a compound that enhances phosphorylation of AMPK for producing a perfusate or preservation solution for a human or animal organ, tissue, or part thereof, wherein the perfusate or the preservation solution is a perfusate or preservation solution for inhibiting injury during preservation, storage, or transportation of an ex vivo organ or tissue, for inhibiting reperfusion injury, for inhibiting graft ischemic injury, for improving functional recovery after transplant of an organ or tissue, or for inhibiting transplant failure.

12. A method of preserving, storing, or transporting an ex vivo human or animal organ, tissue, or part thereof, or a method for inhibiting injury during preservation, storage, or transportation thereof, the method comprising contacting the organ, tissue, or part thereof with a compound that enhances phosphorylation of AMPK.

13. A method of transplanting an ex vivo human or animal organ, tissue, or part thereof,
the method comprising contacting the organ, tissue, or part thereof with a compound that enhances phosphorylation of AMPK before, during, or after transplant thereof.

14. The method according to claim 12 or 13, wherein the contacting the organ, tissue, or part thereof with the compound that enhances phosphorylation of AMPK is selected from the group consisting of perfusion, dipping, rinsing, injection, and combinations thereof.

15. A compound represented by General Formula (III) below or a prodrug thereof, or a pharmaceutically acceptable salt thereof 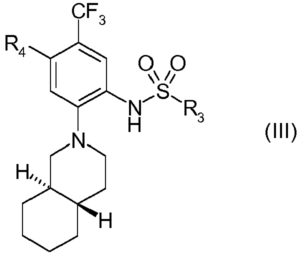 where R₃ represents a substituted or unsubstituted C₁₋₆ alkyl group, substituted or unsubstituted C₂₋₆ alkenyl group, substituted or unsubstituted C₆₋₁₀ aryl group, substituted or unsubstituted nitrogen-containing heterocyclic ring, or substituted or unsubstituted fused aromatic heterocyclic ring, and R₄ represents a hydrogen atom or a halogen atom.

16. A pharmaceutical composition, for inducing programmed cell death in cancer cells, the pharmaceutical composition comprising, as an active component, a compound that enhances phosphorylation of AMPK.

17. The pharmaceutical composition according to claim 16, wherein the enhancement of phosphorylation of AMPK is an enhancement of phosphorylation of AMPK mediated by a G protein-coupled receptor (GPCR).

18. The pharmaceutical composition according to claim 16 or 17, wherein the compound is a compound represented by General Formula (I) below or a prodrug thereof, or a pharmaceutically acceptable salt thereof 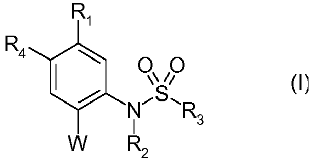
where R₁ represents a hydrogen atom, substituted or unsubstituted C₁₋₆ alkyl group, substituted or unsubstituted C₂₋₆ alkenyl group, substituted or unsubstituted C₂₋₆ alkynyl group, substituted or unsubstituted C₆₋₁₀ aryl group, halogen atom, nitro group, cyano group, azide group, hydroxy group, substituted or unsubstituted C₁₋₆ alkoxy group, substituted or unsubstituted C₁₋₆ alkylthio group, substituted or unsubstituted C₁₋₆ alkylsulfonyl group, carboxy group, formyl group, substituted or unsubstituted C₁₋₆ alkoxycarbonyl group, acyl group, acylamino group, or sulfamoyl group;
R₂ represents a hydrogen atom, substituted or unsubstituted C₁₋₆ alkyl group, or substituted or unsubstituted aryl group;
R₃ represents a substituted or unsubstituted C₁₋₆ alkyl group, substituted or unsubstituted C₂₋₆ alkenyl group, substituted or unsubstituted C₆₋₁₀ aryl group, substituted or unsubstituted nitrogen-containing heterocyclic ring, or substituted or unsubstituted fused aromatic heterocyclic ring;
R₄ represents a hydrogen atom or a halogen atom; and
W represents a hydrogen atom, substituted or unsubstituted C₁₋₆ alkyl group, substituted or unsubstituted C₆₋₁₀ aryl group, halogen atom, hydroxy group, substituted or unsubstituted C₁₋₆ alkoxy group, substituted or unsubstituted C₁₋₆ alkylthio group, substituted or unsubstituted nitrogen-containing heterocyclic ring, or substituted or unsubstituted fused aromatic heterocyclic ring, or W is represented by General Formula (II) below: 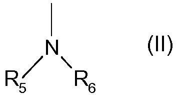
where R₅ and R₆, which are identical to or different from each other, each represent a hydrogen atom, substituted or unsubstituted C₁₋₆ alkyl group, substituted or unsubstituted nitrogen-containing heterocyclic ring, substituted or unsubstituted fused aromatic heterocyclic ring, acyl group, or acylamino group; or
R₅ and R₆ together with an adjacent nitrogen atom each form a substituted or unsubstituted heterocyclic ring or a substituted or unsubstituted fused aromatic heterocyclic ring; or
R₅ and R₆ each represent a substituted or unsubstituted cycloalkylideneamino group or a substituted or unsubstituted aromatic ring-fused cycloalkylidene group.

19. The pharmaceutical composition according to any one of claims 16 to 18, wherein the compound is a compound represented by General Formula (III) below or a prodrug thereof, or a pharmaceutically acceptable salt thereof 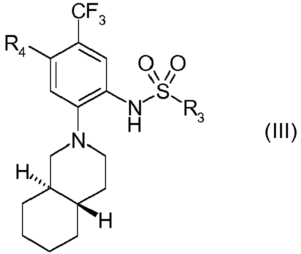 where R₃ represents a substituted or unsubstituted C₁₋₆ alkyl group, substituted or unsubstituted C₂₋₆ alkenyl group, substituted or unsubstituted C₆₋₁₀ aryl group, substituted or unsubstituted nitrogen-containing heterocyclic ring, or substituted or unsubstituted fused aromatic heterocyclic ring, and R₄ represents a hydrogen atom or a halogen atom.

20. The pharmaceutical composition according to any one of claims 16 to 19, wherein the compound is selected from the group consisting of 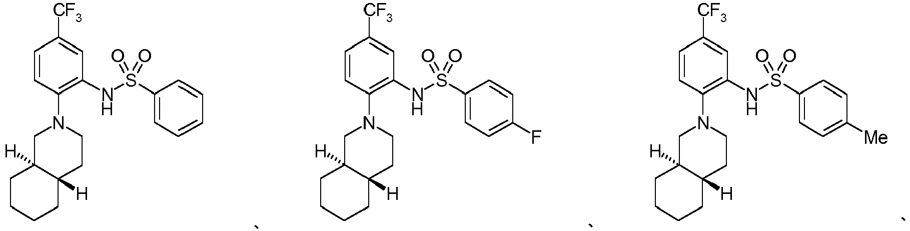 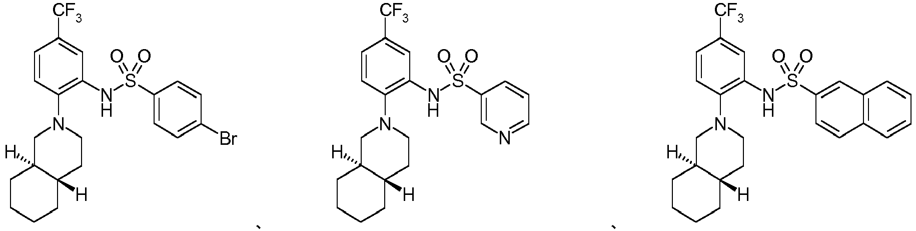 and combinations thereof.

21. A method of cancer prevention, amelioration, progression inhibition, and/or treatment, the method comprising administering a pharmaceutical composition according to any one of claims 16 to 20 to a subject.

22. A method of inducing programmed cell death in cancer cells, the method comprising contacting the cancer cells with a compound represented by General Formula (I) below or a prodrug thereof, or a pharmaceutically acceptable salt thereof, or administering, to a biological body with the cancer cells, a compound represented by General Formula (I) below or a prodrug thereof, or a pharmaceutically acceptable salt thereof 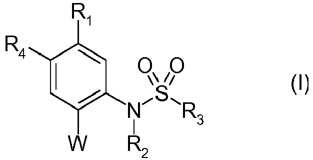
where R₁ represents a hydrogen atom, substituted or unsubstituted C₁₋₆ alkyl group, substituted or unsubstituted C₂₋₆ alkenyl group, substituted or unsubstituted C₂₋₆ alkynyl group, substituted or unsubstituted C₆₋₁₀ aryl group, halogen atom, nitro group, cyano group, azide group, hydroxy group, substituted or unsubstituted C₁₋₆ alkoxy group, substituted or unsubstituted C₁₋₆ alkylthio group, substituted or unsubstituted C₁₋₆ alkylsulfonyl group, carboxy group, formyl group, substituted or unsubstituted C₁₋₆ alkoxycarbonyl group, acyl group, acylamino group, or sulfamoyl group;
R₂ represents a hydrogen atom, substituted or unsubstituted C₁₋₆ alkyl group, or substituted or unsubstituted aryl group;
R₃ represents a substituted or unsubstituted C₁₋₆ alkyl group, substituted or unsubstituted C₂₋₆ alkenyl group, substituted or unsubstituted C₆₋₁₀ aryl group, substituted or unsubstituted nitrogen-containing heterocyclic ring, or substituted or unsubstituted fused aromatic heterocyclic ring;
R₄ represents a hydrogen atom or a halogen atom; and
W represents a hydrogen atom, substituted or unsubstituted C₁₋₆ alkyl group, substituted or unsubstituted C₆₋₁₀ aryl group, halogen atom, hydroxy group, substituted or unsubstituted C₁₋₆ alkoxy group, substituted or unsubstituted C₁₋₆ alkylthio group, substituted or unsubstituted nitrogen-containing heterocyclic ring, or substituted or unsubstituted fused aromatic heterocyclic ring, or W is represented by General Formula (II) below: 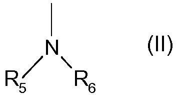
where R₅ and R₆, which are identical to or different from each other, each represent a hydrogen atom, substituted or unsubstituted C₁₋₆ alkyl group, substituted or unsubstituted nitrogen-containing heterocyclic ring, substituted or unsubstituted fused aromatic heterocyclic ring, acyl group, or acylamino group; or
R₅ and R₆ together with an adjacent nitrogen atom each form a substituted or unsubstituted heterocyclic ring or a substituted or unsubstituted fused aromatic heterocyclic ring; or
R₅ and R₆ each represent a substituted or unsubstituted cycloalkylideneamino group or a substituted or unsubstituted aromatic ring-fused cycloalkylidene group.
